# EUROPEAN PATENT APPLICATION

(11) **EP 1 391 463 A1**
(43) Date of publication of application: **25.02.2004**
(21) Application number: 02017524.6
(22) Date of filing: 06.08.2002
(51) Int. Cl.: C07K 14/47

(54) **GPI anchored glycoprotein ACA as a novel tumor marker**

(71) Applicant: Becker-Kojic, Zorica, Dr., 6916 Heidelberg (DE)
(72) Inventor: Becker-Kojic, Zorica, 69126 Heidelberg (DE); Terness, Peter, 69124 Heidelberg (DE)
(74) Representative: Schüssler, Andrea, Dr.

(57) **Abstract**

Described is a GPI-anchored surface glycoprotein having a GPI-anchor which is characterized by a non-acetylated inositol ring and diacyl glycerol as lipid tail of the anchor. A particular glycoprotein of the invention, ACA, is strongly expressed in melanoma cells and some leukemia cells, thus, is an useful marker for diagnosis of said tumors. The invention also relates to salts, functional derivatives and active fractions of ACA having substantially the same spectrum of biological activities of ACA. The invention also relates to a process for the purification of ACA, to its cloning and its production by recombinant DNA techniques. It further relates to diagnostic compositions comprising an anti-ACA-antibody or an oliognucleotide probe capable of hybridizing to ACA-mRNA and to pharmaceutical compounds containing a compound capable of reducing the expression of ACA or the activity of the protein.

## Description

The present invention relates to a GPI-anchored surface glycoprotein having a GPI-anchor which is characterized by a non-acetylated inositol ring and diacyl glycerol as lipid tail of the anchor. A particular glycoprotein of the invention, ACA, is strongly expressed in melanoma cells, some leukemia cells and other tumor cells, thus, is an useful marker for diagnosis of said tumors. The invention also relates to salts, functional derivatives and active fractions of ACA having substantially the same spectrum of biological activities of ACA. The invention also relates to a process for the purification of ACA, to its cloning and its production by recombinant DNA techniques. It further relates to diagnostic compositions comprising an anti-ACA-antibody or an oliognucleotide probe capable of hybridizing to ACA-mRNA and to pharmaceutical compounds containing a compound capable of reducing the expression of ACA or the activity of the protein.

Tumorigenesis represents a complex multistage process in which genetic changes and environmental factors are thought to deregulate the cellular processes that control cell proliferation and differentiation. Melanoma are one of the most common cancers worldwide and the mortality of melanoma patients has increased for 230% in the last years. Diagnosis and monitoring of this type of cancer is difficult because of the heterogeneity of the disease. For diagnosis different grades of malignancy can be distinguished according to the Gleason-Score Diagnosis. For this diagnosis a tissue sample is taken from the patient by biopsy and the morphology of the tissue is investigated. However, this approach only yields subjective results depending on the experience of the pathologist. To summarize, unfortunately, the diagnostic methods used so far are quite insensitive and often yield false-positive results due to lack of specificity. Moreover, by using the current diagnostic methods any conclusions as regards the grade of malignancy, the progression of the tumor and its potential for metastasizing cannot be drawn. Thus, the use of reliable diagnostic molecular markers would be helpful for an understanding of the molecular basis of tumors, e.g. melanomas, for distinguishing benign from malign tissue and for grading and staging carcinoma, particularly for patients with metastasizing melanoma having a very bad prognosis. It would be very useful to have a diagnostic marker in particular for micrometastases in melanoma, which provides information about the metastasis pattern in single tumor patients. This would improve the diagnosis and allow the selection of optimal chemotherapy. Such a marker has not been described so far. It can be expected that such markers are also useful for the development of novel therapeutic avenues for cancer treatment.

Thus, the technical problem underlying the present invention is to provide means for the diagnosis of cancer which overcome the disadvantages of the prior art diagnostic methods.

The solution to said technical problem is achieved by providing the embodiments characterized in the claims.

A method has been elaborated to isolate and purify up to homogeneity, a novel membrane glycoprotein containing a GPI anchor, by means of salting out with ammonium sulfate (40-80% saturation), followed by preparative SDS-PAGE, chromatography and acetone precipitation. The preparation obtained, was homogeneous during electrophoresis in the presence of 0,1% sodium dodecylsulfate after reduction with 2-mercaptoethanol. It is a protein soluble at its isoelectrical point (pH 5,5) with molecular weight of approx. 65.000 Dalton. The isolated protein is linked to the membrane via glycosyl-phosphatidylinositol susceptible to cleavage by purified phospholipase C. The hydrophobic portion of the glycolipid membrane anchor of protein was radiolabeled with the photoactivated reagent 3-(Trifluoromethyl)-3-(m-[¹²⁵J]iodophenyl)diazirine and hydrolyzed with glycosyl-phospatidylinositol specific phospholipase C (GPI-PLC), followed by enzymatic deacetylation of the remaining lipid. Thin-layer chromatography shows that the generated radiolabeled fragment migrates with the same mobility as that of variant surface glycoprotein (VSG), obtained in the same manner. The anchor of ACA - in contrast to other GPI-linked erythrocyte proteins - has a non-acetylated inositol ring and diacyl glycerol, rather than alkyl-acyl glycerol as lipid tail of anchor. Moreover, the distribution and membrane anchoring of ACA was investigated. Rabbit polyclonal antibodies as well as mouse monoclonal antibodies to the purified protein were produced and used to analyze the expression of ACA on human peripheral blood cells by one- and two-color flow cytometry. The results showed that this protein is unimodally present on all granulocytes, monocytes and B lymphocytes, but not on T-cells. Flow-cytometry analysis of erythrocytes showed no measurable protein expression indicating an extremely low abundance of the molecule, whereas highly sensitive immunoblot analysis revealed reactions of both, polyclonal and monoclonal antibodies with the erythrocyte membrane. ACA was removed from the granulocyte membrane by treatment with PI-PLC, an enzyme known to specifically hydrolyze the glycosylphosphatidylinositol (GPI) anchor. The precipitated soluble form of ACA in the supernatant of granulocytes was immuno-blotted and detected with anti-ACA antibodies. B-cells were less susceptible to PI-PLC digestion probably due to partial acetylation of the inositol ring. Epstein-Barr virus (EBV)-transformed B-cell line established from a paroxysmal nocturnal hemoglobinuria (PNH) patient, a disease characterized by partial or absolute deficiency of all proteins anchored to the membrane via inositolglycan, was ACA-negative, in contrast to EBV cells of healthy donors. Finally it was found that ACA is strongly expressed in melanoma and some leukemia cells strongly suggesting important regulatory functions of this protein. Two different monoclonal anti-ACA-antibodies were produced with both of them shown to be useful for the detection of primary and metastatic melanoma cells in immunohistochemistry.

### Figure legends

### Figure 1: Identification of products of phospholipases action

Enzymatic reaction leading to the total hydrolysis of lipid tail of glycosylphosphatidyl inositol of ACA and enzymatic reactions for determinations of glycerol concentration.

### Figure 2: Purification and isolation of ACA

Electrophoresis of erythrocyte ghost ammonium sulfate protein fraction and purified ACA was performed on 4-15% SDS-PAGE. **(A)** lane 1, 2, 3, purified ACA protein obtained from various batches of erythrocytes, lane 4, 5, 40% ammonium sulfate precipitated protein extracts obtained from various batches of erythrocyte membrane, lane 6, molecular weight marker. **(B)** purified ACA protein, has a molecular mass of approx. 65 kD

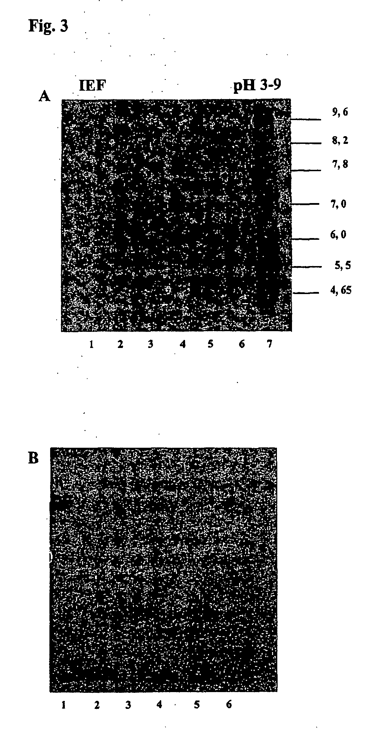

### Figure 3: Isoelectrical focussing of ACA

Electrophoresis of purified ACA on IEF-gradient gel was performed. **(A)** Lines 1-6: purified ACA obtained from various batches of erythrocytes starting from anode, **(B)** starting from cathode. Lane 7A: IEF broad pI calibration standards.

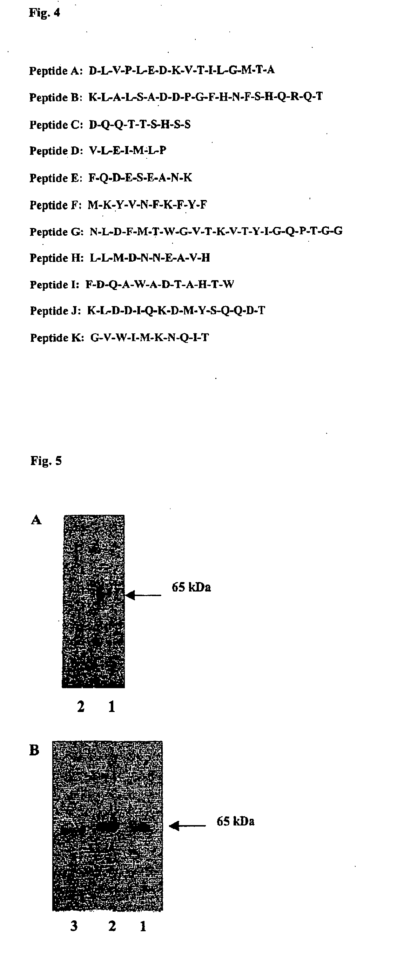

### Figure 4: Sequence of tryptic peptides of ACA

### Figure 5: SDS-PAGE analysis of phospholipases digested proteins

Cleavage of purified ACA with GPI-PLC, PI-PLC and GPI-PLD **(A)** 1, purified ACA; 2, PI-PLC treated ACA **(B)** 1, purified ACA; 2, GPI-PLD treated ACA; 3, ACA treated with GPI-PLC.

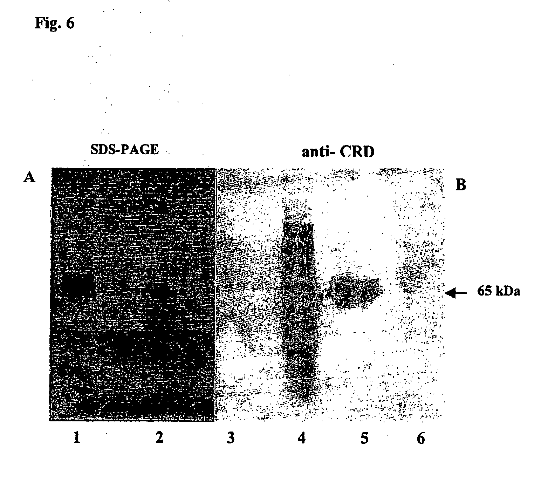

### Figure 6:" Cross-reacting determinant" epitope on ACA

The cross-reacting determinant (CRD) is generated by the action of G/ PI-PLC. **(A)** SDS-PAGE 1, purified native ACA protein; 2, purified ACA GPI-PLC cleaved. (**B**) Immunoblotting with anti-CRD antibody; 3, mf ACA (native protein); 4, GPI-PLC digested form of variant surface glycoprotein (sVSG); 5, ACA digested with GPI-PLC; 6, ACA digested with GPI-PLD.

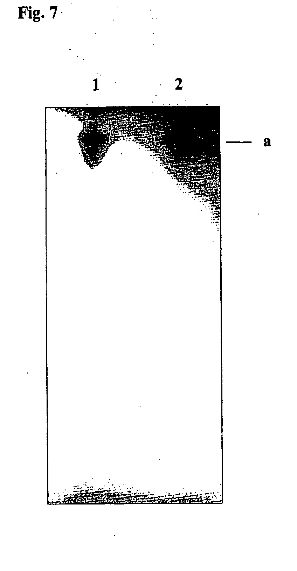

### Figure 7: Silica TLC analysis of fragments generated by hydrolysis of anchors

Samples of [¹²⁵J] TID labeled, purified proteins were hydrolyzed with GPI-PLC. The lipid products of this reaction were further hydrolyzed with highly specific lipases. Radiolabeled fragments were extracted and analyzed by TLC. Myristic acid was used as standard. Lane 1, commercially available mf VSG used as control was labeled, digested with GPI-PLC and further hydrolyzed as described for ACA; lane 2, ACA protein GPI digested and further hydrolyzed .

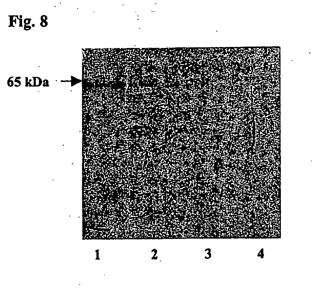

### Figure 8: Western blotting with anti-ACA polyclonal antibody.

Proteins were subjected to SDS-PAGE under reducing conditions, transferred to nitrocellulose and revealed with mouse antibody to ACA. Lane 1, crude protein extract obtained from erythrocyte membrane; lane 2, purified erythrocyte ACA protein; lane 3, erythrocyte ghosts; lane 4, ghosts prepared from erythroleukemia cells.

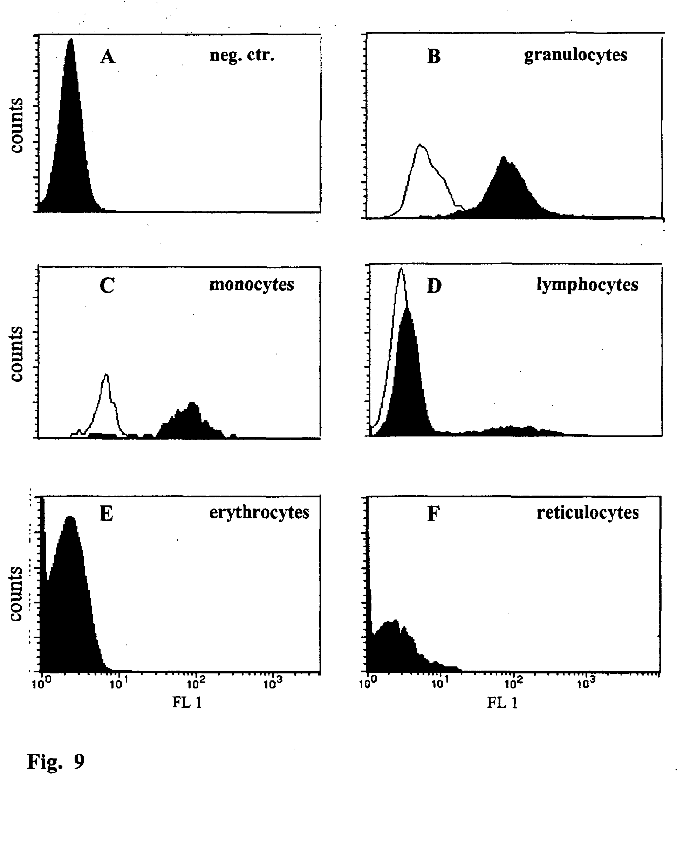

### Fig. 9: Flow cytometric analysis of ACA expression on human peripheral blood cell populations.

Peripheral blood cells of a healthy donor were incubated with mouse monoclonal antibodies to ACA plus anti-mouse-IgG-FITC. After lysis of erythrocytes the granulocytes, monocytes, lymphocytes, and reticulocytes were gated by FSC/SSC. Erythrocytes were separately analyzed. The findings show expression of ACA on granulocytes (b), monocytes (c), and partially on lymphocytes (d). No expression on erythrocytes (e) and reticulocytes (f) was detected. A negative control with an irrelevant control antibody (a) was taken as reference.

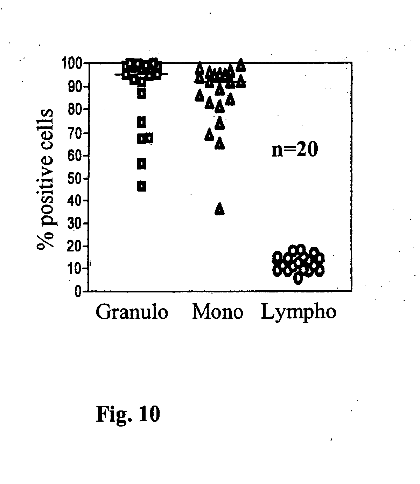

### Fig. 10: Distribution of ACA antigen on peripheral blood cells in healthy donors.

This figure presents the summary of data obtained from analysis of ACA expression on peripheral blood cells of 20 healthy donors as studied by flow cytometry with specific mouse monoclonal antibodies. The expression is shown as percentage of positive cells for each subpopulation: granulocytes, monocytes, and lymphocytes.

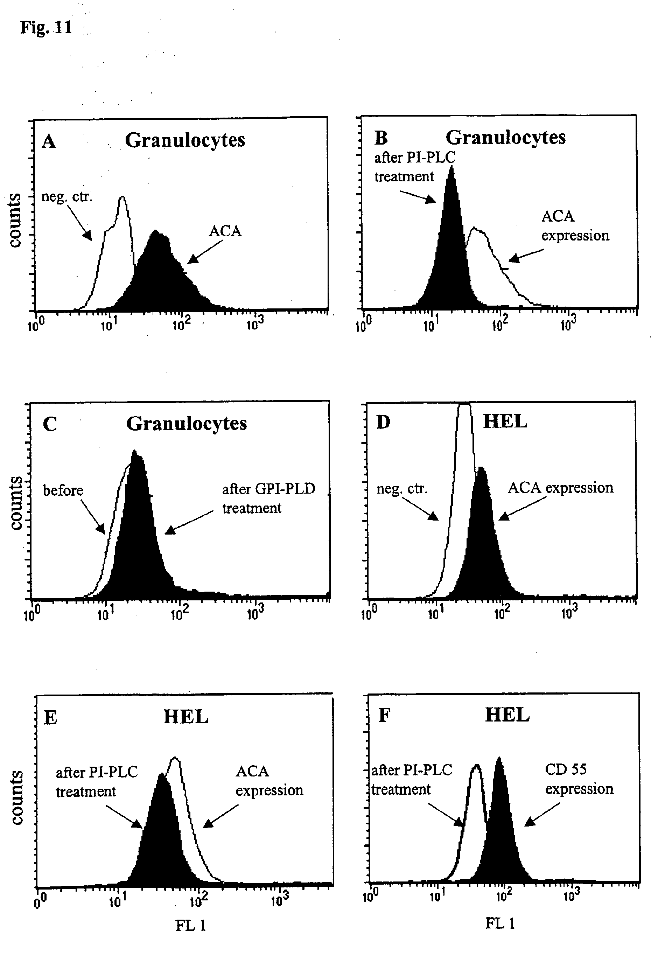

### Fig. 11: Removal of granulocyte membrane-bound ACA by treatment with phosphatidylinositol specific phospholipase C.

Purified granulocytes were treated with buffer alone or with PI-PLC from *Bacillus cereus,* stained with anti-ACA antibodies or irrelevant IgG (negative control) and analyzed as described. Control experiments were done under the same conditions using erythroleukemia (HEL) cells stained with anti-ACA and anti-CD55 antibodies. Another control was performed with bovine serum GPI-phospholipase D, an enzyme which is not able to solubilize GPI-anchored proteins. Histograms show: ACA expression on granulocytes (a), abolished ACA expression on granulocytes after digestion with PI-PLC (b), ACA expression on granulocytes after digestion with GPI-PLD (c), ACA expression on HEL cells (d), abrogation of ACA expression on HEL cells by treatment with PI-PLC (e), abrogation of CD55 expression on HEL cells by treatment with PI-PLC (f).

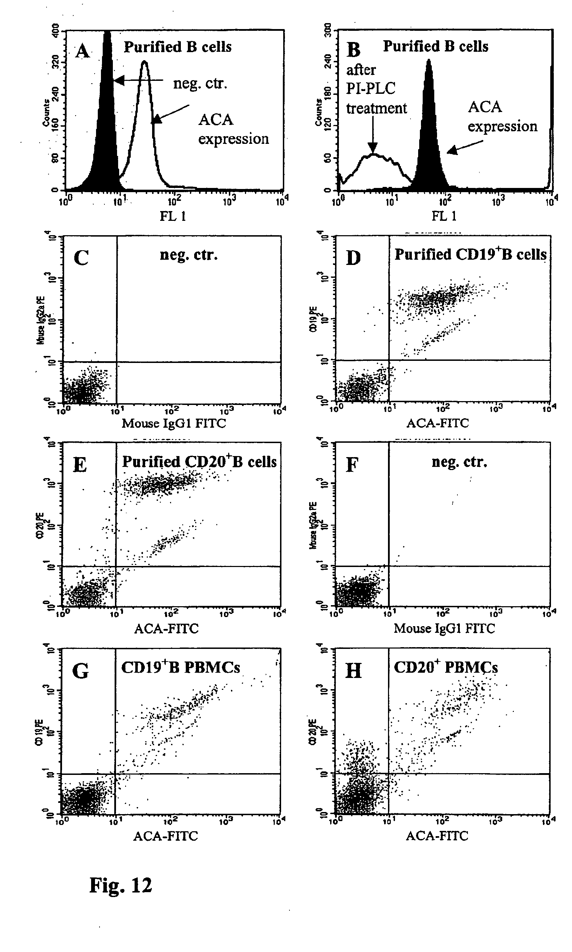

### Fig. 12: Expression of ACA on B lymphocytes and its cleavage by phosphatidylinositol specific phospholipase C.

Purified peripheral B lymphocytes were incubated with elevated concentrations of bacterial PI-PLC as described, stained with the monoclonal antibody to ACA and analyzed by flow cytometry. The histograms show: ACA expression on B lymphocytes and the IgG matched negative control (a), ACA expression after treatment with PI-PLC (b). In a second experiment purified B lymphocytes were incubated with antibodies to ACA (FITC labeling) and to typical B cell markers (CD19, CD20)(PE labeling). The dot-blot analyses show: isotype control (c), ACA expression on CD19- (d) and CD20-positive cells (e). In a third experiment PBMCs were labeled with anti-ACA (FITC labeling) and anti-CD19, -CD20 antibody (PE labeling). The dot blot analyses show isotype control (f), ACA expression on peripheral CD19 positive cells (g), ACA expression on peripheral CD20 positive cells (h). A percentage of 10-15% of PBMC were positive for both ACA and B-cell antigens.

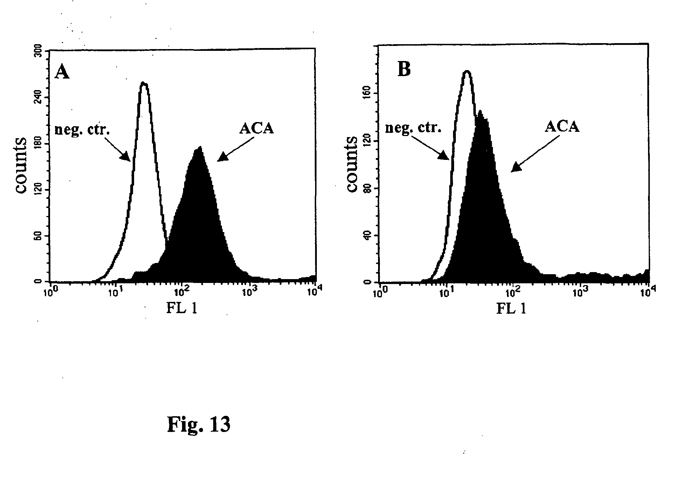

### Fig. 13: Expression of ACA on normal and EBV-transformed PNH B lymphocytes.

EBV-transformed normal and PNH B lymphocytes were generated as described, stained with anti-ACA antibodies (FITC labeling) and analyzed by flow cytometry. Staining with an irrelevant control antibody was taken as reference (neg. ctr.). The histograms show: ACA expression on EBV-transformed B lymphocytes derived from a healthy donor (a), ACA expression on EBV-transformed B lymphocytes derived from a PNH patient (b). Whereas normal B-cells express high levels of ACA, PNH B-cells show significantly reduced expression of the antigen.

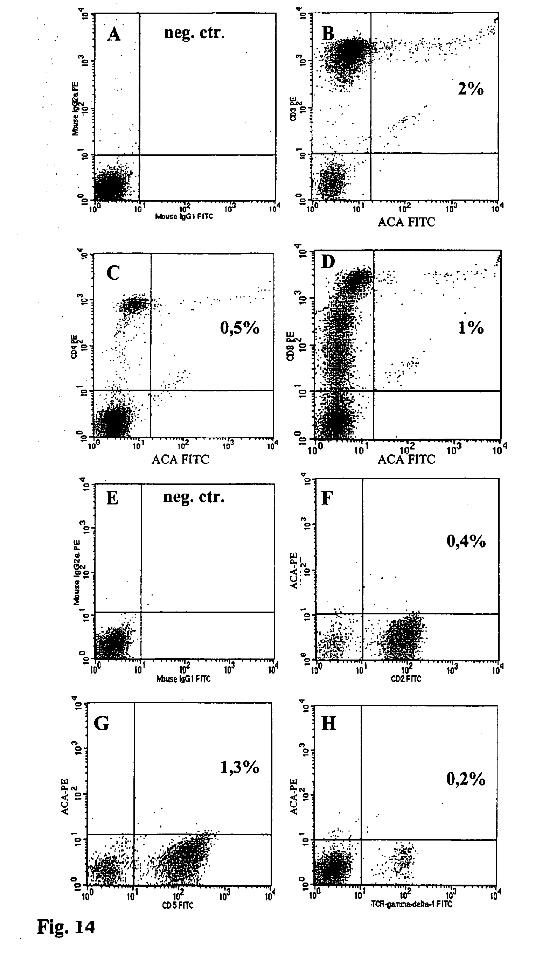

### Fig. 14: Expression of ACA on T lymphocytes.

T lymphocytes were purified as described and stained with monoclonal antibodies to ACA (FITC labeling) and to CD3 (b), CD4 (c), or CD8 (d) (PE labeling). Negative control (a) consisted of irrelevant PE- and FITC-conjugated immunoglobulins. Percentage of ACA-positive cells are shown in the UR quadrant. In a second experiment PBMC were stained with antibodies to ACA (PE-labeling) and to T-cell markers (FITC-labeling) : CD2 (f), CD5 (g), γδ-TCR (h). Negative control (e) consisted of irrelevant PE- and FITC-conjugated immunoglobulins. ACA positive T cells are shown in the UR quadrant.

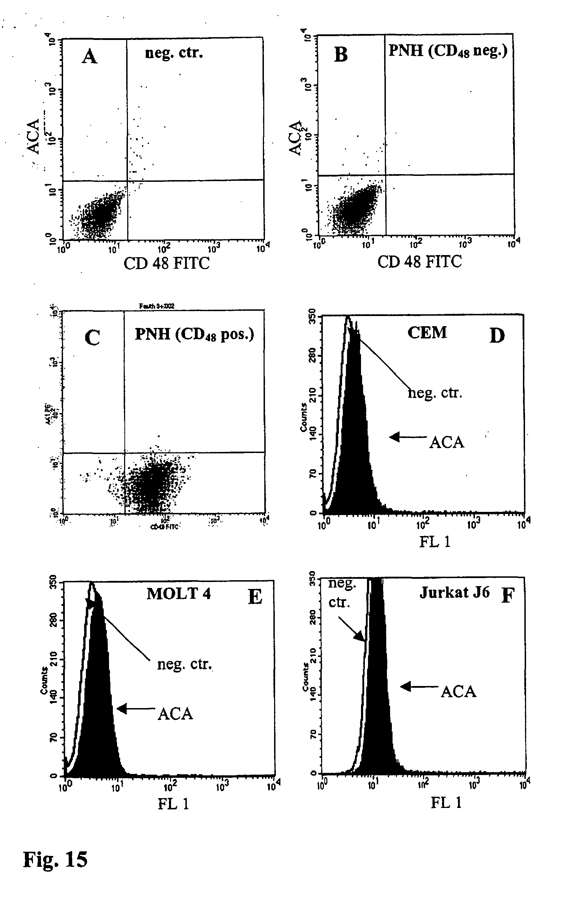

### Fig. 15: ACA expression on T-cell clones derived from patients with PNH or malignancies.

T cell clones obtained from PNH patients, negative (b) or positive (c) for CD48 (=GPI-linked protein) were stained with antibodies to CD48 (FITC labeling) and ACA (PE labeling). The negative control consisted of staining with irrelevant PE- and FITC-conjugated immunoglobulins (a). In an additional experiment cell lines derived from T-cell malignancies were analyzed in one-color flow cytometry using anti-ACA antibodies (FITC labeling). In the negative control the cells were stained with nonreactive FITC-conjugated immunoglobulins. Histograms show ACA expression on CEM cells (d), MOLT4 cells (e), and Jurkat cells (f).

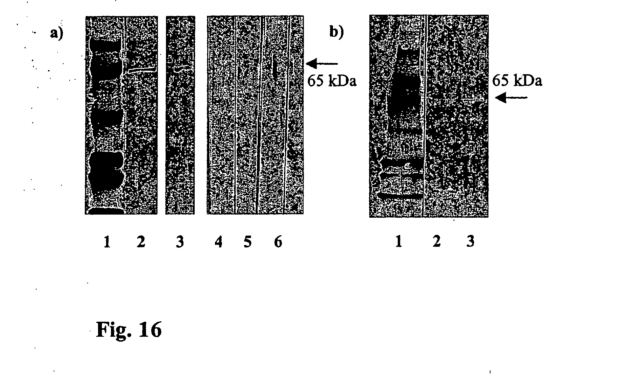

### Fig. 16: Immunodetection of ACA solubilized from peripheral blood cell subpopulations.

Samples of 2x10⁶ cells dissolved as described (a) or supernatant of granulocyte cultures (b) were subjected to SDS-PAGE and proteins were transferred to nitrocellulose. ACA was detected using antibodies previously raised to the purified human erythrocyte ACA protein. Blot membranes were stained with AP-conjugated secondary antibodies and BCIP/NTB substrate. The findings show (a) molecular weight marker (MWM) (lane 1), solubilized B cells (lane 2), solubilized granulocytes (lane 3), solubilized erythrocytes (lane 4), solubilized erythrocyte ghosts (lane 5), crude protein extract from erythrocyte membranes (lane 6). (b) MWM (lane 1), supernatant of granulocytes before PI-PLC digestion (lane 2), precipitated supernatant of granulocytes after PI-PLC digestion (lane 3).

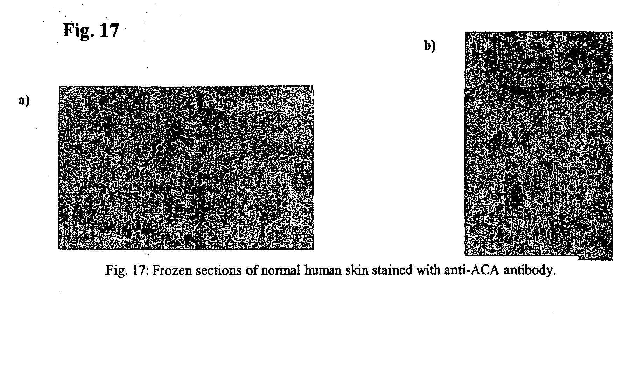

### Figure 17: Frozen sections of normal human skin stained with anti-ACA-antibody.

For details see Examples 1 and 13.

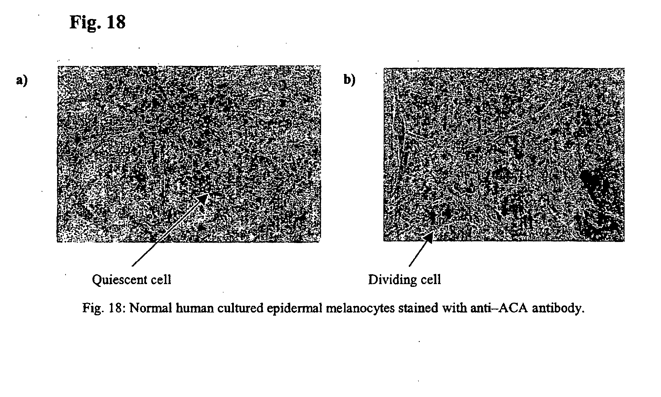

### Figure 18: Normal human cultured epidermal melanocytes stained with anti-ACA-antibody.

For details see Examples 1 and 13.

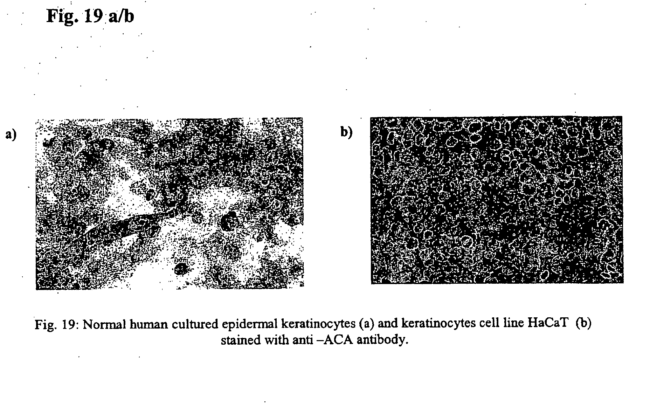

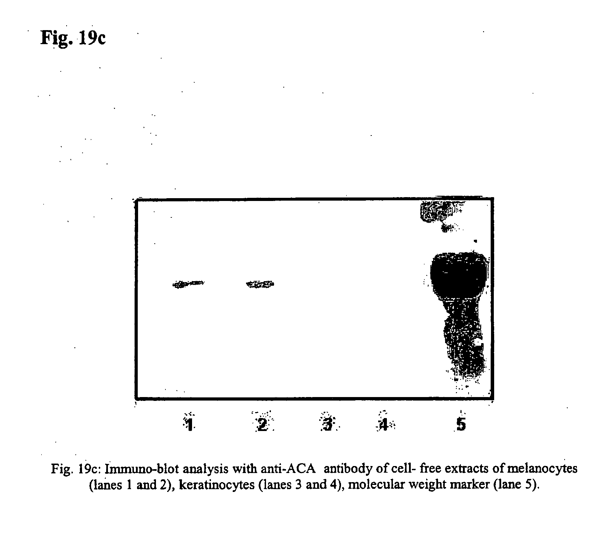

### Figure 19a, b: Normal culture of human epidermal keratinocytes (a) and keratinocyte cell line HaCaT (b) stained with anti-ACA antibody.

For details see Examples 1 and 13.

### Figure 19c: Normal human cultured epidermal keratinocytes (a) and keratinocytes cell line HaCaT (b) stained with anti-ACA-antibody.

Immunoblot analysis with anti-ACA-antibody of cell-free extracts **show:** melanocytes (lanes 1 and 2), keratinocytes (lanes 3 and 4), molecular weight marker (lane 5). For details see Examples 1 and 13.

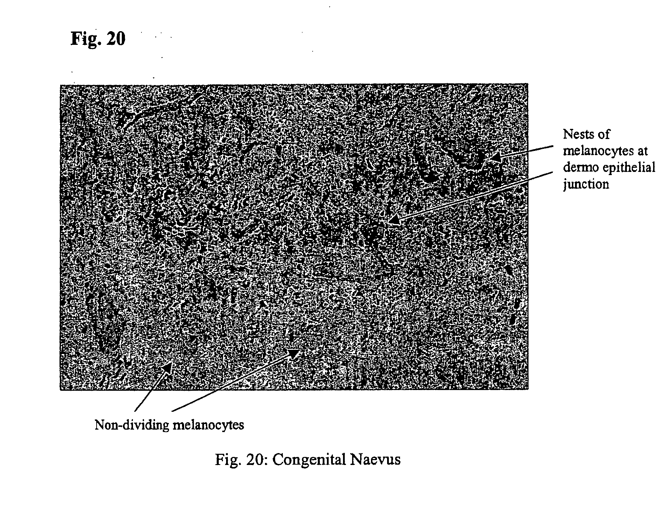

### Figure 20: Congenital Naevus

For details see Examples 1 and 13.

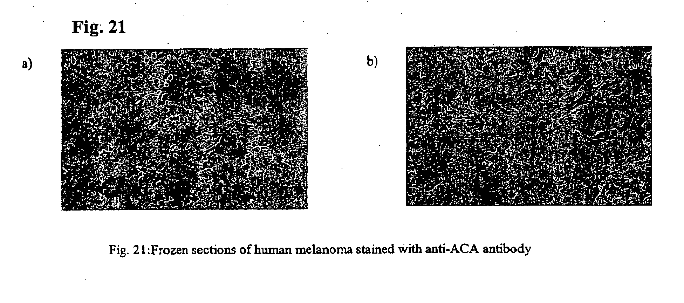

### Figure 21: Frozen sections of human melanoma stained with anti-ACA-antibody

For details see Examples 1 and 13.

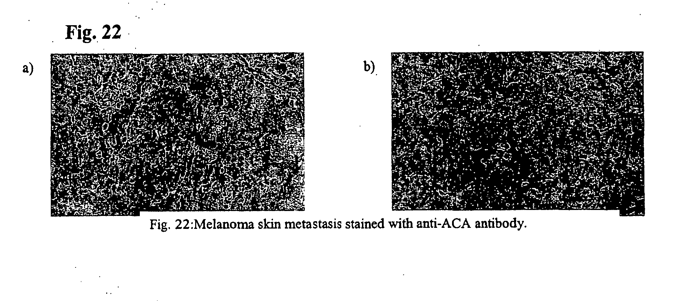

### Figure 22: Melanoma skin metastasis stained with anti-ACA-antibody

For details see Examples 1 and 13.

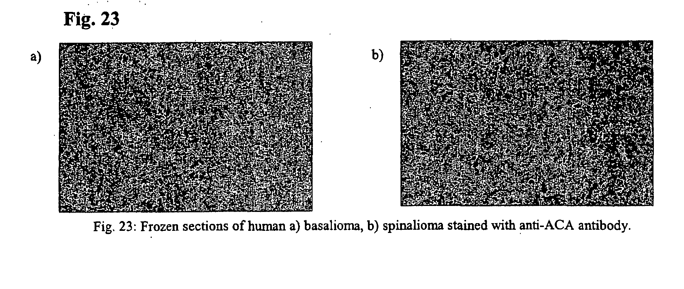

### Figure 23: Frozen sections of human a) basalioma, b) spinalioma stained with anti-ACA-antibody

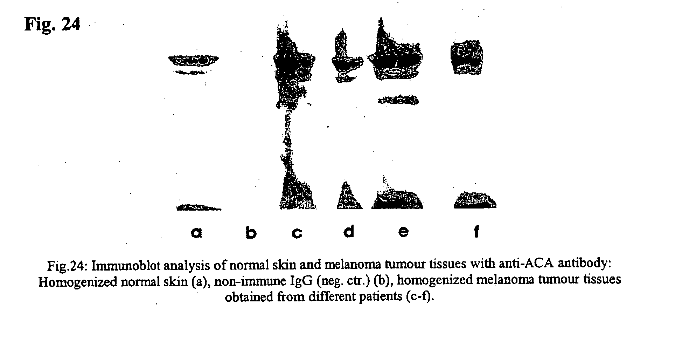

### Figure 24: Immunoblot analysis of normal skin and melanoma tumor tissues stained with anti-ACA-antibody

Homogenized normal skin (a), non-immune IgG (negative control) (b), homogenized melanoma tumor tissues obtained from different patients (c-f)

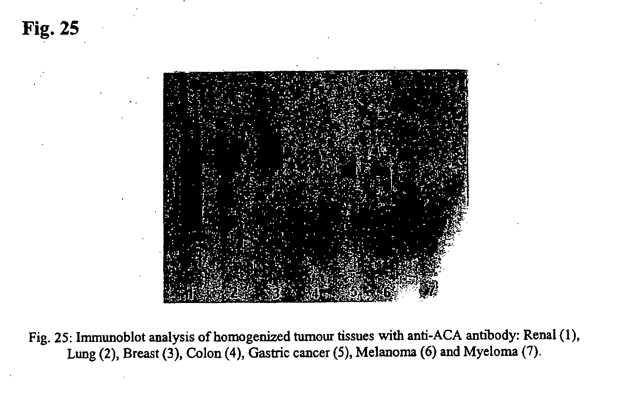

### Figure 25: Immunoblot analysis of homogenized tumor tissues using anti-ACA-antibody

Renal (lane 1), lung (lane 2), breast (lane 3), colon (lane 4), gastric cancer (lane 5), melanoma (lane 6 and myeloma (lane 7).

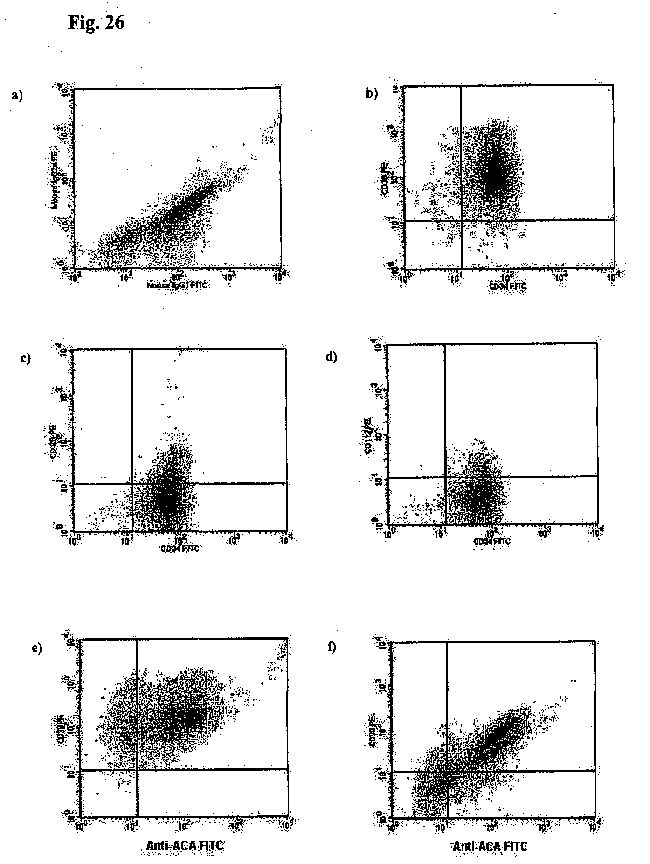

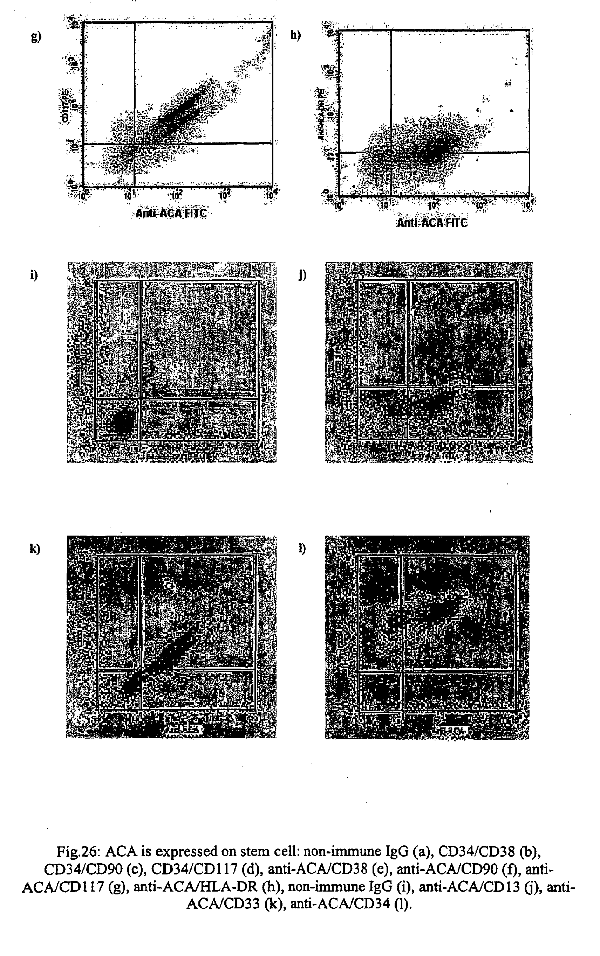

### Figure 26: ACA is expressed on stem cells

Non-immune IgG (a), CD34/CD38 (b), CD34/CD90 (c), CD34/CD117 (d), anti-ACA/CD38 (e), anti-ACA/CD90 (f), anti-ACA/CD117 (g), anti-ACA/HLA-DR (h), non-immune IgG (i), anti-ACA/CD13 (j), anti-ACA/CD33 (k), and anti-ACA/CD34 (1).

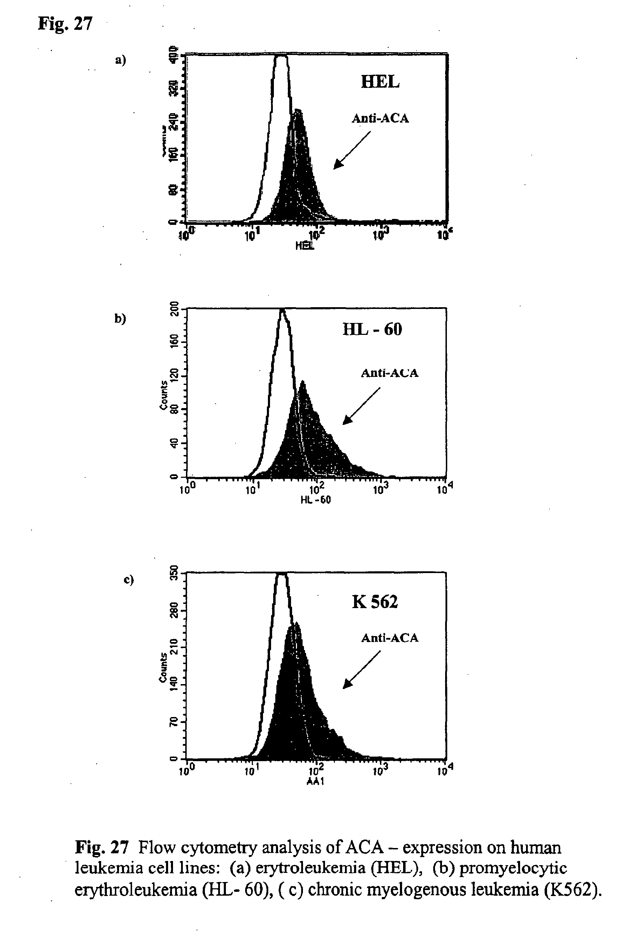

### Figure 27: Representative histograms show ACA expression on human leukemia cell lines

(a) HEL, (b) U-937, (c) HL-60, (d) K-562

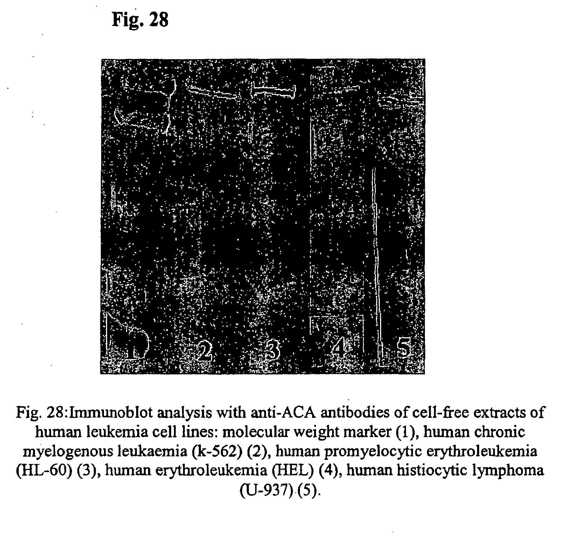

### Figure 28: Immunoblot analysis with anti-ACA-antibodies of cell-free extracts of human leukemia cell lines

(1) Molecular weight marker, (2) human chronic myelogenous leukemia (K-562), (3) human promyelocytic erythroleukemia (HL-60), (4) human erythroleukemia (HEL), (5) human histiocytic lymphoma (U-937)

### Figure 29

**(a, b): Peripheral blood cells of PNH patients were incubated with mouse monoclonal antibodies to ACA plus anti-mouse-IgG FITC and analysed by FACS**
   After lysis of erythrocytes the granulocytes were gated by FSC/SSC.
**(c): Immunoblot analysis with anti-ACA-antibody of granulocytes membrane fraction obtained from healthy donor (lane 1) versus PNH patient (lane 2)**
   lane 3: molecular weight marker.
**(d): Immunoblot analysis with anti-ACA-antibody of PNH granulocytes membrane fraction before (lane 1) and after treatment with phospholipase C (lane 2)**
   lane 3: molecular weight marker.
**(e,f): Immunoblot analysis of soluble form of ACA in supernatants of granulocytes derived from healthy donor (d) and PNH patient (e) after the action of phospholipase C.**

Thus, in one aspect, the present invention relates to a surface glycoprotein having the following features:
(a) it is GPI-anchored on the cell surface;
(b) it can be removed from the cell membrane by treatment with PI-PLC; and
(c) its GPI-anchor is characterized by a non-acetylated inositol ring and diacyl glycerol as lipid tail of the anchor.

Preferably, diacylmyristate or other fatty acids are present in its lipid tail.

In a preferred embodiment, said surface glycoprotein is the surface glycoprotein ACA, characterized by the following additional features:
(a) it has an isoelectric point of pH 5.5;
(b) it is present on blood progenitor cells, granulocytes, monocytes, B-cells (but not T-cells), melanocytes and other cells; and
(c) it is preferentially expressed during cell division and in tumor cells;
or a salt, functional derivative or active fraction thereof.

As used herein the term "salt" refers to both salts of carboxyl groups and to acid addition salts of amino groups of the protein molecule. Since the C-terminus of the protein is attached to the GPI-anchor with diacylmyristate at the end of the molecule, the N-terminus of the protein is blocked, probably acetylated. Therefore, salts cannot be formed at the N-or C-terminus of the native full-length protein. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids such as, for example, hydrochloric acid or sulfuric acid, and salts which organic acids such as, for example, acetic acid or oxalic acid.

"Functional derivative" as used herein covers derivatives having substantially the same biological activity as ACA which may be prepared from the functional groups which occur as side chains an the residues or the N-or C-terminal groups, by means known in the art and are included in the invention as long as they do not destroy the activity of the protein and do not confer toxic properties an compositions containing it. These derivatives may, for example, include aliphatic esters of the carboxyl groups, amides of the carboxyl groups by reaction with ammonia or with primary or secondary amines, derivatives of free amino groups of the amino acid residues formed with acyl moieties (e.g. alkanoyl or carbocyclic aroyl groups) or 0-acyl derivatives of free hydroxyl group (for example that of seryl or threonyl residues) formed with acyl moieties.

An "active fraction" of the ACA of the present invention covers any fragment or precursor of the polypeptide chain of the protein molecule alone or together with associated molecules or residues linked thereto, e.g. sugar or phosphate residues, or aggregates of the protein molecule or the sugar residues by themselves, provided said fraction has at least one biological activity of ACA, e.g. signal transduction activity.

The surface glycoprotein ACA of the present invention, preferably, is obtainable, from human blood by
(a) isolating and lysing cells, preferably erythrocytes;
(b) isolating, disrupting and pelleting the hemoglobin free membrane of said cells;
(c) repeated salting out of the resuspended membranes with ammonium sulfate (70%; 40% saturation);
(d) subjecting the proteins precipitated in step (c) to preparative SDS-PAGE under reducing conditions; and
(e) isolating the gel band of the protein.

The various steps of this method can be carried out according to standard protocols, e.g., the protocols described in the following examples.

In a preferred embodiment, the surface glycoprotein ACA of the present invention is furthermore characterized by a molecular weight of about 65 kD when analyzed by SDS PAGE under reducing conditions.

In an even more preferred embodiment, the surface glycoprotein ACA of the present invention contains at least one of the following amino acid sequences:
(a) D-L-V-P-L-E-D-K-V-T-I-L-G-M-T-A;
(b) K-L-A-L-S-A-D-D-P-G-F-H-N-F-S-H-Q-R-Q-T;
(c) D-Q-Q-T-T-S-H-S-S;
(d) V-L-E-I-M-L-P;
(e) F-Q-D-E-S-E-A-N-K;
(f) M-K-Y-V-N-F-K-F-Y-F;
(g) N-L-D-F-M-T-W-G-V-T-K-V-T-Y-I-G-Q-P-T-G-G;
(h) L-L-M-D-N-N-E-A-V-H;
(i) F-D-Q-A-W-A-D-T-A-H-T-W;
(j) K-L-D-D-I-Q-K-D-M-Y-S-Q-Q-D-T; or
(k) G-V-W-I-M-K-N-Q-I-T.

The protein sequence data for ACA are available in the SWISS-PROT and TrEMBL knowledge base under the accession number 83408.

The present invention also relates to a process for the isolation of a surface glycoprotein ACA which comprises:
a) isolating and lysing cells, preferably erythrocytes, from human blood;
(b) isolating, disrupting and pelleting the hemoglobin free membrane of said cells;
(c) repeated salting out of the resuspended membranes with ammonium sulfate (70%; 40% saturation);
(d) subjecting the proteins precipitated in step (c) to preparative SDS-PAGE under reducing conditions; and
(e) isolating the gel band of a 65 kD protein.

The present invention also relates to a recombinant surface glycoprotein ACA which is, preferably, produced in a mammalian cell. Methods for the recombinant production of ACA using a nucleic acid molecule of the invention are described below.

The present invention further concerns a nucleic acid molecule, preferably a DNA molecule, comprising a nucleotide sequence encoding the surface glycoprotein ACA of the invention or a functional derivative or fragment thereof, wherein said surface glycoprotein ACA contains at least one of the following amino acid sequences:
(a) D-L-V-P-L-E-D-K-V-T-I-L-G-M-T-A;
(b) K-L-A-L-S-A-D-D-P-G-F-H-N-F-S-H-Q-R-Q-T;
(c) D-Q-Q-T-T-S-H-S-S;
(d) V-L-E-I-M-L-P;
(e) F-Q-D-E-S-E-A-N-K;
(f) M-K-Y-V-N-F-K-F-Y-F;
(g) N-L-D-F-M-T-W-G-V-T-K-V-T-Y-I-G-Q-P-T-G-G;
(h) L-L-M-D-N-N-E-A-V-H;
(i) F-D-Q-A-W-A-D-T-A-H-T-W;
(j) K-L-D-D-I-Q-K-D-M-Y-S-Q-Q-D-T; or
(k) G-V-W-I-M-K-N-Q-I-T.

The term "DNA molecule" includes genomic DNA, cDNA, synthetic DNA and combinations thereof.

The cloning of ACA may be carried out by different techniques. According to one approach, specific antibodies (polyclonal or monoclonal) to ACA are produced and used to clone ACA. This approach is based on the following three steps:
(A) Preparation of antibodies: anti-ACA-antibodies can be produced either by using the substantially purified ACA of the present invention or by using one or more synthetic peptides identical to the known amino acid sequence of the protein, or by fusing one of the possible nucleotide sequences deduced from the amino acid sequence of ACA to the gene coding for Protein A and expressing the fused Protein A - ACA in E. coli. The generation of suitable antibodies is described below.
(B) Screening of ACA producing cells: The antibodies to ACA are used to search for cells producing ACA by immunofluorescence or by Western blot. Examples of suitable cells can be found in the Examples, below.
(C) Preparation of cDNA from producing cells: mRNA is extracted from ACA producing cells and cDNA is prepared by the use of reverse transcriptase. The cDNA is cloned in an expression vector such as lambda-gT11, lambda-"ZAP" (e.g., "UNI-ZAP™ XR custom cDNA library"; Stratagene, La Jolla, CA, USA) and screened by the use of the antibodies prepared according to standard methods, e.g. the "SEREX"-method (Sahin et al., Proc. Natl. Acad. Sci. USA, Vol. 92, S. 11810-11813, 1995) or the method described by Türeci et al. (Cancer Res. 56 (1996), 4766-4772). Antibodies binding to recombinant proteins expressed in lytic plaques can be detected, e.g., by incubation with an alkaline phosphatase-conjugated goat anti-mouse IgG (e.g. available from DAKO, Glostrup, Danmark) and visualized by staining with 5-bromo-4-chloro-3-indolyl phosphate and nitroblue tetrazolium.

Following another approach, a synthetic oligonucleotide or a mixture of synthetic oligonucleotide, whose sequence is derived from the sequence of a fragment of the ACA protein are produced and this oligonucleotide or the mixture of oligonucleotides are used as a probe for cloning the cDNA or the genomic DNA coding for the ACA Protein. The genomic DNA may or may not include naturally occurring introns. It may be obtained, for example, by extraction from suitable cells and purification by means well known in the art. Suitable DNA preparations, such as human genomic DNA, are enzymatically cleaved by restriction enzymes, or randomly sheared, and the fragments inserted into appropriate recombinant vectors to form a gene library. Such vectors can then be screened with synthetic oligonucleotides probes in order to identify a sequence coding for the ACA protein of the present invention.

Alternatively, mRNA is isolated from a cell which expresses the ACA protein of the invention and used to produce cDNA by means well known in the art; see, e.g. Sambrook et al., 1989, Molecular Cloning: A Laboratory Manual, 2^{nd} edition, Cold Spring Harbor Laboratory Press, NY, USA. This cDNA, after conversion to the double-stranded form, may be cloned and the resulting clone screened with an appropriate probe for cDNA coding for the desired sequences. Once the desired clone has been isolated, the cDNA may be manipulated in substantially the same manner as the genomic DNA. However, with cDNA there will be no introns or intervening sequences. In order to design the oligonucleotides to be used as probes, one can use the partial amino acid sequences shown in Figure 4. In addition, it is possible either to perform sequence analysis of the intact ACA protein or to obtain further peptide fragments thereof and to characterize their amino acid sequence. In order to obtain peptide fragments, purified protein preparations are subjected to fragmentation, e.g. by digestion with proteases such as trypsin, chymotrypsin or papain by methods well known in the art, e.g. the methods described in the examples, below. The peptide fragments produced by digestion are separated by reversed phase HPLC and sequenced by automatic amino acid sequencing techniques.

Once one or more suitable peptide fragments have been sequenced or a partial sequence of the protein is determined, the DNA sequences capable of encoding them are examined. Due to the degeneration of the genetic code, more than one codon may be used to encode a particular amino acid and one or more different oligonucleotides can be produced, each of which would be capable of encoding the ACA protein peptide fragments. However, only one member of the set contains the nucleotide sequence that is identical to the nucleotide sequence of the gene. Its presence within the set and its capability to hybridize to DNA even in the presence of the other members of the set, makes it possible to employ the unfractionated set of oligonucleotides in the same manner in which one would employ a single oligonucleotide to clone the gene that encodes the peptide. The use of such oligonucleotide or set of oligonucleotides containing the theoretical "most probable" sequence capable of encoding the ACA Protein gene fragments (following the "codon usage rules" disclosed in standard literature) permits to identify the sequence of a complementary oligonucleotide or set oligonucleotides which is capable of hybridizing to the "most probable" sequence encoding the ACA protein or at least a portion thereof, or a set of such sequences. This oligonucleotide containing such a complementary sequence may then be synthesized and employed as a probe to identify and isolate the gene of the ACA protein of the invention. Once a suitable oligonucleotide, or set of oligonucleotides, which is capable of encoding a fragment of the ACA protein gene (or which is complementary to such an oligonucleotide, or set of oligonucleotides) is identified using the above-described procedure, it is synthesized and hybridized to a DNA or preferably, to a cDNA preparation derived from cells which are capable of expressing the desired gene, preferably after the source of cDNA has been enriched for the desired sequences, e.g. by extracting RNA from cells which produce high levels of the desired gene and then converting it to the corresponding cDNA by employing the enzyme reverse transcriptase. Procedures for hybridization of nucleic acids are common knowledge. By hybridization whith the above oligonucleotides or set of oligonucleotides probes, it is possible to identify in a cDNA or genomic library, the DNA sequences capable of such hybridization and they are then analyzed to determine to what extent they contain encoding sequences for the ACA protein of the invention.

Alternatively, primers for a PCR reaction can be designed on the basis of a partial amino acid sequence of ACA and used to isolate the gene encoding ACA or a part thereof using as a source the cells or libraries described above. Finally, on the basis of the nucleic acid sequences derived from the partial amino acid sequences of ACA commonly available gene banks, e.g. EST based gene banks, can be screened.

The invention furthermore relates to vectors containing the nucleic acid molecules of the invention. Preferably, they are plasmids, cosmids, viruses, bacteriophages and other vectors usually used in the field of genetic engineering. Vectors suitable for use in the present invention include, but are not limited to the T7-based expression vector for expression in bacteria, the pMSXND expression vector for expression in mammalian cells and baculovirus-derived vectors for expression in insect cells. Preferably, the nucleic acid molecule of the invention is operatively linked to the regulatory elements in the recombinant vector of the invention that guarantee the transcription and synthesis of an RNA in prokaryotic and/or eukaryotic cells that can be translated. The nucleotide sequence to be transcribed can be operably linked to a promotor like a T7, metallothionein I or polyhedrin promoter.

In a further embodiment, the present invention relates to recombinant host cells transiently or stably containing the nucleic acid molecules or vectors of the invention. A host cell is understood to be an organism that is capable to take up *in vitro* recombinant DNA and, if the case may be, to synthesize the ACA proteins, fragments etc. encoded by the nucleic acid molecules of the invention. Preferably, these cells are prokaryotic or eukaryotic cells, for example mammalian cells, bacterial cells, insect cells or yeast cells. The host cells of the invention are preferably characterized by the fact that the introduced nucleic acid molecule of the invention either is heterologous with regard to the transformed cell, i.e. that it does not naturally occur in these cells, or is localized at a place in the genome different from that of the corresponding naturally occurring sequence.

A further embodiment of the invention relates to a method for the recombinant production of an ACA protein of the invention, whereby, e.g. a host cell of the invention is cultivated under conditions allowing the synthesis of the protein and the protein is subsequently isolated from the cultivated cells and/or the culture medium. Isolation and purification of the recombinantly produced proteins may be carried out by conventional means including preparative chromatography and affinity and immunological separations involving affinity chromatography with monoclonal or polyclonal anti-ACA-antibodies. The ACA proteins are preferably in a substantially purified form. A recombinantly produced version of ACA can be substantially purified by the one-step method described in Smith and Johnson, Gene 67:31-40 (1988).

The present invention also relates to an antibody to the surface glycoprotein ACA of the present invention. The term "antibody", preferably, relates to antibodies which consist essentially of pooled monoclonal antibodies with different epitopic specificities, as well as distinct monoclonal antibody preparations. Monoclonal antibodies are made from an antigen containing fragments of the proteins of the invention by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fv, Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a Fab or other immunoglobulin expression library. Moreover, antibodies of the present invention include chimerical, single chain, and humanized antibodies.

The present invention also relates to a method for the diagnosis of a tumor associated with overexpression of ACA or a predisposition for such a tumor which comprises contacting a target sample with a compound which is capable of (i) specifically binding to the surface glycoprotein ACA according to the present invention or (ii) an mRNA transcribed from the ACA encoding DNA molecule and (b) comparing the level of ACA protein or ACA-mRNA of the sample determined by use of the above compound with a control sample obtained from a healthy individual, wherein an elevated level of the surface glycoprotein ACA or the corresponding mRNA is indicative for a tumor or a predisposition for such a tumor. Preferably, said compound is an anti-ACA-antibody or an oligonucleotide which is capable of hybridizing to an mRNA transcribed from a DNA sequence encoding ACA. Preferably, the nucleic acid sequence of said oligonucleotide hybridizing with ACA DNA has a length of at least 12, preferably of at least 15, or, more preferably, of at least 20 bases. The oligonucleotides used as probes can be detectably labeled, for example, with a radioisotope, a bioluminescent compound, a chemiluminescent compound, a fluorescent compound, a metal chelate, or an enzyme.

The target cellular component, ACA protein or ACA-mRNA, in biological fluids, e.g. blood, or tissues, may be detected directly in situ, e.g. by in situ hybridization or it may be isolated from other cell components by common methods known to those skilled in the art before contacting with a probe. Detection methods include Northern blot analysis, RNase protection, in situ methods, e.g. in situ hybridization, in vitro amplification methods (PCR, LCR, QRNA replicase or RNA-transcription/amplification (TAS, 3SR), reverse dot blot disclosed in EP-B1 0 237 362)), immunoassays, Western blot and other detection assays that are known to those skilled in the art.

Products obtained by in vitro amplification can be detected according to established methods, e.g. by separating the products on agarose gels and by subsequent staining with ethidium bromide. Alternatively, the amplified products can be detected by using labeled primers for amplification or labeled dNTPs.

Expression of ACA in tissues can be studied with classical immunohistological methods (Jalkanen et al., J. Cell. Biol. 101 (1985), 976-985; Jalkanen et al., J. Cell. Biol. 105 (1987), 3087-3096; Sobol et al. Clin. Immunpathol. 24 (1982), 139-144; Sobol et al., Cancer 65 (1985), 2005-2010). Other antibody based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (¹²⁵I, ¹²¹I), carbon (¹⁴C), sulfur (³⁵S), tritium (³H), indium (¹¹²In), and technetium (⁹⁹mTc), and fluorescent labels, such as fluorescein and rhodamine, and biotin. In addition to assaying ACA levels in a biological sample, the protein can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma. A protein-specific antibody or antibody fragment, which has been labeled with an appropriate detectable imaging moiety, such as a radioisotope (for example, ¹³¹I, ¹¹²In, ⁹⁹mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of ⁹⁹mTc. The labeled antibody or antibody fragment will then preferentially accumulate at the location of cells, which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982)).

It can be concluded that by inhibiting the expression and/or activity of ACA an effective therapy of cancers like melanoma is provided.

Thus, the present invention also relates to a pharmaceutical composition containing a compound capable of reducing or eliminating (a) the expression of the nucleic acid sequence encoding the surface glycoprotein ACA and/or (b) the biological activity of ACA. Examples of such reagents are antisense RNAs, ribozymes or inhibitors of the biological activity of the protein, e.g. specific antibodies. For example, administration of an antibody directed to the protein can bind and reduce overproduction of the protein.

An antisense RNA sequence according to the present invention is characterized in that it is complementary to an mRNA transcribed from a nucleic acid molecule of the present invention or a part thereof and can selectively bind to said mRNA, said sequence being capable of inhibiting the synthesis of the ACA protein encoded by said nucleic acid molecules, and a ribozyme characterized in that it is complementary to an mRNA transcribed from a nucleic acid molecule of the present invention or a part thereof and can selectively bind to and cleave said mRNA, thus inhibiting the synthesis of the ACA protein encoded by said nucleic acid molecules. Ribozymes, which are composed of a single RNA chain are RNA enzymes, i.e. catalytic RNAs, which can intermolecularly cleave a target RNA, for example the mRNA transcribed from one of the Trp genes. It is now possible to construct ribozymes, which are able to cleave the target RNA at a specific site by following the strategies described in the literature. (see, e.g., Tanner et al., in: Antisense Research and Applications, CRC Press Inc. (1993), 415-426). The two main requirements for such ribozymes are the catalytic domain and regions which are complementary to the target RNA and which allow them to bind to its substrate, which is a prerequisite for cleavage. Said complementary sequences, i.e., the antisense RNA or ribozyme, are useful for repression of ACA-expression, e.g. in the case of the treatment of a melanoma. Preferably, the antisense RNA and ribozyme of the invention are complementary to the coding region of the mRNA, e.g. to the 5' part of the coding region. The person skilled in the art provided with the sequences of the nucleic acid molecules of the present invention will be in a position to produce and utilize the above described antisense RNAs or ribozymes. The region of the antisense RNA and ribozyme, respectively, which shows complementarity to the mRNA transcribed from the nucleic acid molecules of the present invention preferably has a length of at least 10, in particular of at least 15 and particularly preferred of at least 50 nucleotides.

Inhibitors for the ACA protein can be, for instance, structural analogues of the corresponding protein that act as antagonists. In addition, such inhibitors comprise molecules identified by the use of the recombinantly produced proteins, e.g. the recombinantly produced protein can be used to screen for and identify inhibitors, for example, by exploiting the capability of potential inhibitors to bind to the protein under appropriate conditions. The inhibitors can, for example, be identified by preparing a test mixture wherein the inhibitor candidate is incubated with the ACA protein under appropriate conditions that allow ACA to be in a native conformation. Such an in vitro test system can be established according to methods well known in the art. Inhibitors can be identified, for example, by first screening for either synthetic or naturally occurring molecules that bind to the recombinantly produced ACA protein and then, in a second step, by testing those selected molecules in cellular assays for inhibition of the ACA protein, as reflected by inhibition of at least one of the biological activities. Such screening for molecules that bind the ACA protein could easily performed on a large scale, e.g. by screening candidate molecules from libraries of synthetic and/or natural molecules. Such an inhibitor is, e.g., a synthetic organic chemical, a natural fermentation product, a substance extracted from a microorganism, plant or animal, or a peptide. A particular function of these inhibitors is to block a cell-to cell transfer of ACA. Additional examples of inhibitors are specific antibodies, preferably monoclonal antibodies. Moreover, the nucleic sequences of the invention and the encoded proteins can be used to identify further factors involved in tumor development and progression. Moreover, the proteins of the invention can, e.g., be used to identify further (unrelated) proteins which are associated with, e.g., a melanoma using screening methods based on protein/protein interactions, e.g. the two-hybrid-system.

For administration the above compounds are preferably combined with suitable pharmaceutical carriers. Examples of suitable pharmaceutical carriers are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the tumor and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the tumor, general health and other drugs being administered concurrently.

The delivery of the antisense RNAs or ribozymes of the invention can be achieved by direct application or, preferably, by using a recombinant expression vector such as a chimeric virus containing these compounds or a colloidal dispersion system. By delivering these nucleic acids to the desired target, the intracellular expression of ACA and, thus, the level of ACA can be decreased resulting in the inhibition of the negative effects of ACA, e.g. as regards the metastasis formation of a melanoma.

Direct application to the target site can be performed, e.g., by ballistic delivery, as a colloidal dispersion system or by catheter to a site in artery. The colloidal dispersion systems which can be used for delivery of the above nucleic acids include macromolecule complexes, nanocapsules, microspheres, beads and lipid-based systems including oil-in-water emulsions, (mixed) micelles, liposomes and lipoplexes. The preferred colloidal system is a liposome. The composition of the liposome is usually a combination of phospholipids and steroids, especially cholesterol. The skilled person is in a position to select such liposomes which are suitable for the delivery of the desired nucleic acid molecule. Organ-specific or cell-specific liposomes can be used in order to achieve delivery only to the desired tumor. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific tumors via specific cell-surface ligands.

Preferred recombinant vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

In order to achieve expression only in the target organ, i.e. tumor to be treated, the nucleic acids encoding, e.g. an antisense RNA or ribozyme can also be operably linked to a tissue specific promoter and used for gene therapy. Such promoters are well known to those skilled in the art (see e.g. Zimmermann et al, (1994) Neuron 12, 11-24; Vidal et al., (1990) EMBO J. 9, 833-840; Mayford et al., (1995), Cell 81, 891-904; Pinkert et al., (1987) Genes & Dev. 1, 268-76).

For use in the diagnostic research discussed above, kits are also provided by the present invention. Such kits are useful for the detection of a target cellular component, which is ACA or, alternatively, ACA encoding mRNA. Said kits comprise a probe for detection of ACA or, alternatively, ACA encoding mRNA. The probe can be detectably labeled. Such probe may be a specific antibody or specific oligonucleotide. Preferably, the nucleic acid sequence of said oligonucleotide hybridizing with ACA DNA has a length of at least 12, preferably of at least 15, or, more preferably, of at least 20 bases. In a preferred embodiment, said kit contains an anti-ACA-antibody and allows said diagnosis, e.g., by ELISA and contains the antibody bound to a solid support, for example, a polystyrene microtiter dish or nitrocellulose paper, using techniques known in the art. Alternatively, said kits are based on a RIA and contain said antibody marked with a radioactive isotope. In a preferred embodiment of the kit of the invention the antibody is labeled with enzymes, fluorescent compounds, luminescent compounds, ferromagnetic probes or radioactive compounds.

The following Examples illustrate the invention.

### Example 1: General methods

### (A) Purification and isolation of protein

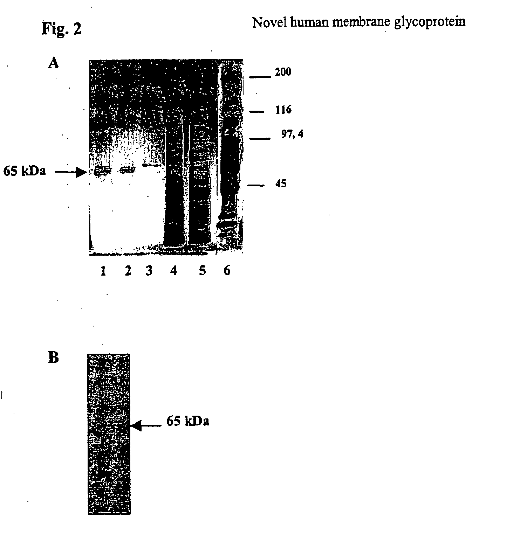

Human blood withdrawn from healthy donor was centrifuged at 1500 g, at 4°C for 10 min. Plasma and buffy coat were removed by aspiration and packed cells were washed twice in 150mM / 15mM sodium phosphate (pH 7,6) and lysed in CH₃ COOH / distilled water. Membranes were pelleted at 10. 000 x g for 15 min. and washed with extraction buffer, 50mM Tris-HCl pH 7.5, 2mM DTT, 1mM PMSF, 1mM EDTA, 0,25M Sucrose. This step was repeated until the ghosts showed no visual evidence of residual hemoglobin. (Three washes were usually required). After the last wash, the ghosts were resuspended in the same buffer. The pellet was frozen in fluid nitrogen for 15 min and thaws at 25°C. This procedure is repeated for three times. The homogenate was then adjusted to 400 mM with respect to KCl and centrifuged at 25.000 g for 60 min. The supernatant was collected and adjusted to 70% saturation with (NH₄)₂SO₄ (Sigma, München, Germany) and stirred for 30 min. The precipitated proteins were collected by centrifugation, redissolved in the extraction buffer and precipitated again with 40% saturation ¹with (NH₄)₂SO₄ ( Fig. 2A). The precipitated proteins are stirred again for 30 min on ice, redissolved in extraction buffer and dialyzed against storage buffer, (40mM Tris-HCl pH 7.5, 2mM DTT, 1mM EDTA, 0.25 M sucrose), and stored at -20°C. The pure amphiphilic form of protein was obtained by further preparative steps including SDS gel electrophoresis. The gel bands of the protein were excised, crushed in 25mM Tris-base, 192 mM glycine and 0,035% SDS (pH 8,3) and the supernatant was collected after overnight incubation at 4°C. Purified protein was dialyzed against the storage buffer (NH₄)₂CO₃, precipitated with ice cold acetone in ethanol/dry ice bath and stored at 4°C. The protein concentrations were measured by the methods of Bradford (Anal. Biochem. 72 (1976), 284-254).

### (B) Electrophoretic methods

SDS-PAGE was performed on linear gradient gel of 4-15% polyacrylamide in the discontinuous buffer of Laemmli (Laemmli, Nature 227 (1970), 680-685). The samples were boiled for 2 min. in the reducing sample buffer before electrophoresis. Protein bands were identified by silver staining, or negative staining by CuCl₂. Molecular weights were estimated by comparison with the position of molecular weight standards purchased from Bio-Rad Laboratories (München, Germany). Gels were fixed with methanol/acetic acid or ethanol/acetic acid and visualized with Coomassie blue or by standards silver staining.

### (C) Isoelectrical focussing

A total of 1 to 5 ng of protein per band was subjected to PhastGel IEF gradient media supplied by Pharmacia (Freiburg, Germany). The samples were applied approximately 10 mm from cathode. Additional controls were done applying samples next to anode and cathode. The bands were visualized using the silver staining method according to manufacturer's instruction. For standards, the Pharmacia Broad pI Calibration KIT on PhastGel IEF 3-9 was used.

### (D) Chemical deglycosylation

Chemical deglycosylation was performed in order to prepare ACA protein for primary structural analysis. Protein sample free of salts, metal ions and detergents was treated with anhydrous trifluoromethanesulfonic acid (TMSF) (Oxford GlycoScience, UK) for 4.5 h in a freezer, neutralised and recovered in 0,5% ammonium bicarbonate. The neutralized reaction mixture was then dialysed against the same buffer and precipitated deglycosylated protein isolated directly by centrifugation.

### (E) Protein sequence analysis

Chemically deglycosylated protein was treated with trypsin or Asp-N at 37° C for 18 h. Peptides were separated by C₁₈ reverse-phase HPLC. N-terminal sequences were determined by automated Edman degradation using gas-phase sequencer.

### (F) Cell-free extracts

Preparation of ghosts was done according to protocol approved by the Institutional Review Board of the New York Blood Center. Briefly, about 2,3x10⁶ / ml packed red cells and erythroleukemia cells (K-562) were washed once with PBS buffer at 5000 rpm for 5 min. After the supernatant was decanted, the cells were lysed with 1:20,5 dilution of PBS in distilled water, centrifuge at 15.000 rpm for 10 min. Supernatant was decanted and the discrete pellet of the white cells adhering to the centrifuge tube was removed too (Dodge et al., Arch. Biochem. Biophys. 100 (1963), 119-130). Resuspended ghosts were washed with 10 mM Tris.HCl (pH 7.8) until they become white and fluffy. Equal volume of simple buffer was added and boiled for 2 min. before subjected to SDS-PAGE using 4,5 stacking gel and 4-15% separating gel.

Alternatively, blood cells (2x10⁶) in PBS were washed twice with the same volume of PBS at 5000 rpm for 5 min. The pellet was dissolved in PBS/water (1:20.5), incubated on ice for 10 min., and centrifuged to remove insoluble material. The supernatant was collected and stored. The remaining pellet was extracted with 10mM Tris-HCl (pH 7.8) and centrifuged at 15000g for 15 min. The supernatant was then analyzed in SDS-PAGE and immuno-blotting. The proteins released from the membrane of granulocytes after the treatment of cells with PI-PLC, were precipitated by adding 5 volume of ice cold acetone and incubation in a dry ice ethanol bath for 30 min. The precipitated protein was centrifuged at 13500g for 10 min., acetone carefully removed from the pellet, and the pellet was air dried until the residual acetone evaporated. The recovered protein was analyzed in SDS-PAGE and immunoblotting.

To prepare membrane protein fractions from normal skin and melanoma tumor tissue simples, 20 frozen sections a 20 mµ were incubated on ice, in buffer A (50 mM Tris.HCl, pH 7,5, 25 mm KCl, 3 mM MgCl₂ 0,25 M Saccharose, 1 mM PMSF, 2 mM Dithiotheitol, 1 mM EDTA) for 30 min, and homogenized in Ultra Turrax T8 machine. The extracts were centrifuged 1300 rpm 10 min at 4°C to remove insoluble material, and the supernatants were collected and stored. The remaining pellet was extracted once more with buffer A, centrifuged and added to the previous supernatants. The protein in supernatants was precipitated with 5X volume of ice-cold methanol and samples centrifuged at 13.500 rpm. The precipitated proteins were dissolved in buffer B (40mM Tris.HCl, pH 7,5, 2 mM DTT, 1 mM EDTA and 0,25 Saccharose) and the protein concentration determined according to method of Bradford.

### (G) Immunoblotting

Membrane proteins (10-30 µg) prepared from the erythrocyte ghosts and homogenized tissue of erythroleukemia (K-652) cells, were mixed with reducing sample buffer and subjected to sodium dodecyl-sulfate/polyacrylamide gel electrophoresis (SDS-PAGE) and than transferred to nitrocellulose membrane. The filters were blocked with 3% BSA in PBS and used for testing antibody reactivity. Polyclonal mouse serum against 65 kD GPI-protein, was used at dilution 1: 5000. Visualization was performed using anti-IgG secondary antibody, conjugated with alkaline phosphatase (AP) and BCIP/NTB as a chromogenic substrate for AP (Promega, USA).

Alternatively, cell-free extracts and supernatant after PI-PLC digestion were subjected to SDS-PAGE under reducing conditions on a 4-15% gradient gel and then blotted onto nitrocellulose. Nitrocellulose membranes were blocked with TBS containing 0.1% Tween-20 and 3% BSA, followed by 1h incubation with primary antibody. The antibodies were used at the following concentrations: monoclonal mouse antibodies (AC1, AB12) at the dilution 1:1000, polyclonal rabbit antibody (AL1) at the dilution 1:500. The secondary antibodies, alkaline phosphatase-conjugated goat anti-mouse and goat anti-rabbit IgG (Promega, Heidelberg, Germany) were used at a dilution of 1:10 000. 5-Bromo-4-chloro-indolyl phosphate tetrazolium (BCIP/NTB) liquid substrate system (Boehringer Mannheim GmbH, Germany) was used as a chromogenic substrate for alkaline phosphatase.

### (H) Digestions with phospholipases

Digestion with glycosyl phosphatidylinositol-phospholipase C (GPI-PLC) from *Trypanosoma brucei* (Oxford GlycoScience, UK), was performed for 1h at 30°C. Samples of proteins were further buffered by addition of TBS (Tris-buffered saline, 20 mM Tris-HCl, 0,15 M NaCl, containing 0,1% of Triton X-114, 10 mM TLCK and 5 mM EDTA) and incubated with 5 µl (25 U/ml) enzyme. Incubations with 5 mU of phosphatidyl inositol specific phospholipase C, (PI-PLC) *(Bacillus cereus,* Boehringer Mannheim, Germany) were performed in triethanolamine buffer (50 mM triethanolamine, 10 mM EDTA, and 10 mM sodium azide, pH 7.5) for 1h at 37°C. Digestion with anchor specific phospholipase D (from bovine serum EC 3.1.4.50 Boehringer Mannheim) was performed in 20 mM Tris-HCl pH 7.4, 0.1 mM CaCl₂ Triton X-100 0.008% (w/vol.), for 60 min. at 37°C. After the reactions were finished, 10 µl of reducing sample buffer was added. Tubes containing the reaction mixtures were placed in a boiling water bath for 5 minutes, and aliquots of the samples were subsequently subjected to analysis by SDS-PAGE and silver staining (PhastGel System from Pharmacia Biotech, Freiburg, Germany).

Alternatively, digestion assays were performed using 10⁶ purified granulocytes in 25 µl PBS with sequentially diluted (1-5 U/mL) glycosyl phosphatidylinositol specific phospholipase C (GPI-PLC) (*Bacillus thuringiensis,* Oxford GlycoScience, UK), in Tris-HCl buffer (pH 7,4) at 37°C for 1hr. Solubilization with PI-PLC *(Bacillus cereus,* Boehringer Mannheim) was performed with 0,5 U enzyme in 200 µl Tris.HCl buffer, 10 mM EDTA (pH 7,8) at 37°C for 1hr. For the solubilization of ACA, on B lymphocytes, Triton X-100 was added to the latter, at the concentration of 0,03%. Control experiments were done simultaneously on HEL ACA-positive cells, after digestion with *Bacillus cereus* PI-PLC under the same reaction conditions, as those described for blood cells. Digestion with phosphatidylinositol phospholipase D (PI-PLD)(5mU) was performed with purified granulocytes in 20 mmol/l Tris.HCl buffer (pH 7.4), 0,1 mmol/l CaCl₂, 0,008 % Triton X-100, for 60 min at 37°C in 25 µl reaction volume.

### (I) Anti-CRD (cross reacting determinant) Assay

The GPI-PLC, PI-PLC and GPI-PLD cleaved proteins were subjected to SDS-PAGE and transferred onto nitrocellulose. After blocking, the antigens were visualized with affinity purified anti-CRD antibody (Oxford, GlycoScience). After washing, bound anti-CRD was detected by incubation with biotinylated donkey anti-rabbit IgG, and visualized with streptavidin horse radish peroxidase. As positive and negative controls, soluble form of variant surface glycoprotein (sVSG) and membrane form of ACA were blotted simultaneously and revealed with anti-CRD antibody.

### (J) Identification of products of phospholipases action

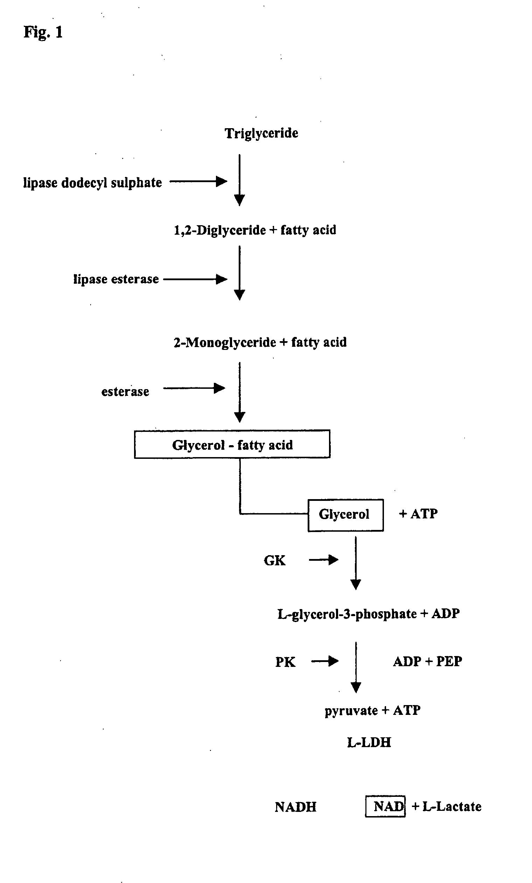

Purified ACA protein was digested with PI- and GPI-phospholipases C, and subjected to further hydrolysis with triacylglycerol acyl hydrolase (300 U) ( *Rhizopus arrhizus* EC 3.1.1.3., Boehringer Mannheim, Germany), followed by carboxylic ester hydrolase (30 U) (from pig liver EC 3.1.1.1., Boehringer Mannheim) in reaction buffer containing, 0.1 mg of sodium dodecyl sulfate/ml, for 25 min at 25°C. Glycerol was determined as described by purchaser (Boehringer Mannheim) with glycerokinase, (GK) pyrivate kinase, (PK) and lactate dehydrogenase (LDH) as auxiliary and indicatory enzymes (Boehringer Mannheim). Lipase, esterase, PK, LDH, GK were free from hexokinase, as well as other interfering kinases and phosphatases, ATP (Boehringer Mannheim) was substantially free from ADP, and phosphoenol pyrivate PEP (Boehringer Mannheim), from pyrivate. The decrease in the extinction at 340 nm, 334 and 365 nm due to oxidation of nicotinamid adenin dinucleotide (NADH) was measured (Figure 1).

### (K) Radiolabeling

Purified proteins were labeled with 3-(trifluoromethyl)-3-(m-[¹²⁵J] iodophenyl) diazirine ([¹²⁵J] TID) (Amersham) by photolysis at 350 nm for 15 min, as described by Roberts and Rosenberry (Biochemistry 25 (1986), 3091-3098). A 10 µl aliquot of [¹²⁵J] TID in ethanol (5-200 µCi) was added to 500 µl of buffered red blood cells ACA protein in 1 cm² rubber-stoppered borosilikate glass test tube, and agitated gently to ensure complete mixing. Photolysis was performed for 20 minutes in Beckman spectrophotometer, with the slits removed.

### (L) Thin-layer chromatography (TLC)

Samples of GPI-PLC and PI-PLC cleaved [¹²⁵J]TID labeled proteins, were subjected to further hydrolysis with triacyl glycero-protein acylhydrolase, followed by carboxylic-ester hydrolase, and the lipid products of this reaction were extracted with chloroform/methanol/ HCl ( 22:50:1), centrifuged for 15 min at 300 g. The lower phase was removed and evaporated to dryness under nitrogen. The residues were resolved in 0.1 ml of chloroform/methanol (2:1) and these solutions were quantitatively applied to TLC plates. Thin-layer chromatography was performed on 10x20 cm Silica Gel plates (Merck, Darmstadt, Germany) without activation. Development was either with solvent B [hexane/diethylether/acetic acid (60:30:1)] or solvent A [hexane/2-propanol, (96:4)]. The positions of radioactive components were identified by autoradiography with Kodak XAR-5 film. The positions of standards were located by exposure of dried plates to iodine vapor.

### (M) Generation of antibodies to ACA

The novel erythrocyte membrane protein ACA was isolated from normal human erythrocytes as described above. Rabbit polyclonal antibody (AL1) was generated by immunization of animals with 100 µg purified protein in complete Freund's adjuvant for the initial inoculation and 50µg antigen mix with incomplete Freund's adjuvant for additional three boosters. The antibody titer was determined by ELISA and immunoblotting with purified protein and cell-free extracts. Two mouse monoclonal antibodies (AC1 and AB12) were generated according to a standard protocol (Fazekas et al., J. Immunol. Methods 35 (1981), 1-21). Briefly, BALB/C mice were immunized 3 times with ACA in Freund's adjuvant. The spleen cells were collected, hybridoma generated and cloned. Antibody binding was verified by ELISA and immunoblot.

### (N) Separation of cells

Heparinized human peripheral blood of 20 healthy donors was used as starting material. Granulocytes were isolated from blood by centrifugation on Ficoll-Hypaque (Pharmacia, Freiburg, Germany) density gradient. Peripheral blood mononuclear cells (PBMC) were removed, and granulocytes sedimented at 2000g after incubation with dextran/phosphate-buffered saline (PBS) solution (Pharmacia), 30 minutes, at room temperature. The contaminating erythrocytes were removed by hypotonic lysis. T and B lymphocytes were separated according to a previously described method (Nicholson-Weller et al., Blood 65 (1985), 1237-1244). Briefly, PBMC isolated through Ficoll-Hypaque density gradient centrifugation, were resuspended in RPMI 1640 medium (GIBCO, Eggenstein, Germany), supplemented with 10% bovine serum albumin (BSA) (GIBCO) and incubated with neuraminidase treated sheep erythrocytes. B and T lymphocytes were separated after Ficoll gradient centrifugation of rosetted lymphocytes. Cells were washed twice with PBS and kept at 4°C until use. Purity of each cell population was >95%. Viability was >95% as determined by vital staining with trypan-blue.

### (O) Generation of human-cell lines for antibody characterization

For the generation of EBV-transformed PNH (GPI-deficient) B lymphocyte lines, Ficoll-Hypaque-separated PBMC were incubated with antibody to CD48(a GPI-anchored leucocyte marker)(Immunotech, Hamburg, Germany), followed by complement lysis with human AB serum (Taylor et al., Biochem. J. 322 (1997), 919-925; Tomita, Biochimica et Biophysica Acta 1455 (1999), 269-286). PNH cells, deficient in CD48 and other GPI-linked surface antigens remain after complement lysis with human AB serum (Taylor et al., 1997). GPI-positive B lymphocytes isolated from normal donors and GPI-negative B lymphocytes of PNH patients were incubated for 3 hours at 37° C with EBV suspension and analyzed by flow cytometry. CD48⁻ and CD48⁺ B-cells were cultured in Dulbecco's modified Eagle medium (DMEM)(GIBCO) supplemented with 20% heat inactivated fetal bovine serum (FBS) and 2mmol/L glutamine. For generation of T-cell lines, homogeneous populations of CD48⁻ and CD48⁺ lymphocytes were obtained by limiting dilution according to standard procedure (Fleisher, J. Immunol. Methods 109 (1988), 215; Hertenstein et al., Blood 86 (1995), 1487-1492). In brief, PBMC from PNH patients were isolated and T lymphocytes separated as described above. Affected GPI⁻ T lymphocytes were enriched as previously described for B lymphocytes. The cells were grown in histoplates (Nunc, Roskilde, Denmark) on irradiated allogenic PBMC as feeding layer and supernatant of phytohemagglutinin-stimulated PBMC. T lymphocytes were seeded at 0,1, 0,3 and 1,0 cell/well. Growing clones were transferred to larger wells and restimulated weekly with irradiated PBMC as already described.¹⁶T-cell clones were cultured in RPMI 1640 medium (GIBCO) supplemented with 7,5% human AS serum and 20 U/mL recombinant human interleukin-2, and analyzed by two color flow cytometry. T-cell lines like MOLT 4, CEM, Jurkat J6 were cultured in RPMI 1640 supplemented with 10% FBS and 2mM/L glutamine. Human erythroleukemia (HEL) cell-line was grown in RPMI 1640 culture medium supplemented with 2 mM glutamine and 10% FBS.

### (P) Flow cytometric analysis

### (i) Blood samples

Flow cytometry for whole blood was performed by using a two-step method as described elsewhere (Schrezenmeier et al., Exp. Hematol. 23 (1995), 81-87). Briefly, to 100 µl of EDTA-blood were added 50 µl PBS with 0,1% BSA and 5% horse serum and incubated 30 min at 37°C. The mixture was then incubated for 20 minutes at room temperature with 2 µl of mouse monoclonal AC1 or AB12 antibody, or with irrelevant isotype control, followed by fluorescein isothiocyanate (FITC)-labeled goat anti-mouse IgG (Dako, Hamburg, Germany). After washing and incubation with 1ml IMMUNO-LYSIS working solution (Coulter Clone, Hialeah, FL) for 2 min, the cells were fixed with 1% paraformaldehyde and analyzed using a FACScan flow cytometer (Becton Dickinson, Heidelberg, Germany). Data were analyzed using LYSIS II software (Becton Dickinson). Fluorescence intensity was presented on a log scale. Granulocytes, monocytes and lymphocytes were identified by characteristic forward and right angle light scattering, gated, and at least 10000 events were analyzed for each sample. For analysis of red cells 2 µl venous blood sample was added to 200 µl PBS/BSA/horse serum reagent and incubated for 30 minutes at 37°C, followed by 20 min incubation with AC1, AB12 or irrelevant monoclonal antibody of the same subclass, as control. After washing, the pellet was resuspended in 200 µl PBS/BSA containing the secondary antibody and after 20 min incubation at room temperature cells were washed three times, and the pellet resuspended in 2 ml PBS with 0,1% BSA and paraformaldehyde added to a final concentration of 2%. Erythrocytes were analyzed without gating by measuring 10000 cells. For analysis of reticulocytes 1ml PBS with 0,1% BSA and 2µl EDTA-blood were incubated with 1ml of thiazol orange solution (Retic-count) (Becton Dickinson) for 20 min at room temperature. Staining of reticulocytes by the RNA dye thiazol orange allowed for a separate analysis of erythrocytes and reticulocytes. Two-color analysis was performed as described above except for the various FITC- or phycoerythrin (PE)-labeled commercially available monoclonal antibodies to specific cell markers (anti-TCR and CD2; Coulter Immunotech, Hamburg, Germany), (CD19, CD20, CD38; Becton Dickinson), (CD5; Dako, Hamburg, Germany), which were used at manufacturer's recommended dilutions as a third step. Color compensation for crossover of fluorescence signals between the two detectors was adjusted for optimal analysis. In both one-color and two-color analysis, the negative fraction was defined as cells having approximately the same low intensity as the negative control population, and the positive fraction was defined as the cell population with intensity similar to that of the positive control population.

### (ii) Isolated blood cells and cell lines

Immunophenotyping of isolated granulocytes, B and T lymphocytes was performed as described above. Briefly, 100 µl of cell suspensions in PBS were incubated with 2 µl of primary antibodies AC1 and AB12 or appropriate dilution of irrelevant monoclonal antibody, in dark at room temperature for 20 minutes, washed and incubated with goat-anti-mouse PE or FITC IgG. Two-color analysis was performed by using directly FITC or PE labeled monoclonal antibodies to specific cell-markers like CD19, CD20, CD3, CD4, CD8, (Becton Dickinson) at recommended dilution as a third step. Color compensation for crossover of fluorescence signals between the two detectors was adjusted for optimal analysis. Immunofluorescence analysis of cell lines was performed after the cells were washed three times with PBS, and resuspended at 10⁶/ml in FACS buffer (1% BSA and 0,2% natriumazide in PBS). All staining steps were conducted on ice. Various cells were incubated with primary antibodies AC1, AB12 (30 µg/ml), or CD55 (Serotec, Wiesbaden, Germany) for 30 min followed by incubation with FITC-conjugated secondary antibodies. Cells were washed three times in FACS buffer and fixed with 1% formaldehyde. Susceptibility to cleavage by PI-PLC was assessed by incubation of cells as described below. Cells were washed, stained as described and analyzed by flow- cytometry.

### (Q) Tumor tissues and cell lines

Representative tissue samples were freshly received from normal skin and cutaneous melanocytic lesions excised from patients at the University of Heidelberg. They were snap frozen in liquid nitrogen and stored at -80°C until sectioning. Five µm cryostat sections were cut from normal skin and comprising different stages of melanocytic tumor progression and different skin tumors air dried overnight at room temperature, and fixed 20 minutes in a Coplin jar containing reagent-grade cold (-20°C) acetone. The slides were than dried 10 minutes at room temperature incubated for 30 min. with PBS, 5% BSA in the staining chamber, to eliminate the unspecific binding, and than incubated with primary antibodies to ACA at dilutions of 1:2000 to 1:4000. After washing the sections were incubated serially with link antibody (biotinylated rabbit anti-mouse immunoglobulin), streptavidin-biotin complex, amplification reagent and streptavidin-peroxidase. Bound monoclonal antibodies were detected with AEC, which give rise to a red chromogen. The sections were counterstained with Harris's hematoxylin and finally mounted in aqueous mounting medium.

Normal human epidermal melanocytes and keratinocytes were purchased from Promo-Cell (Germany) and cultured as monolayer in serum and phorbol-myristat-acetate (PMA) free melanocytes (keratinocytes) growth medium. For the immunohistological staining, cells were grown in special chambers as monolayer. Fixation and staining follow the procedure described above. Human erythroleukemia (HEL), human histiocytic lymphoma (U 937), human promyelocytic leukemia (HL-60) and human chronic myelogenous leukemia (K562) cell-lines were grown in RPMI 1640 culture medium supplemented with 2mM glutamine and 10% FBS. One-color flow cytometry of cell-lines was carried out according to the procedure described above.

### Example 2: Purification of ACA and amino acid sequencing of purified ACA

A two-step purification procedure was applied for the preparation of the protein. The first step includes the disruption of the hemoglobin free membrane followed by repeated precipitation with ammonium sulfate of various saturation grades and preparative electrophoresis (Figure 2A). The purity of preparations was evaluated by SDS-PAGE (Figure 2, A and B) and isoelectrical focussing (IEF), (Figure 3,A and B). Preparations were obtained from various batches of pooled human erythrocytes. No degradation products were identified in any preparation.

Purified and chemically deglycosylated ACA was subjected to N-terminal amino acid sequencing. No sequence was obtained, indicating a blocked N-terminus. Therefore, the protein was inactivated and trypsinized, and sequence was established for 11 internal peptides (Figure 4). Only peptide A shows homology with # 1230 # 1248 region of spectrin.

### Example 3: Digestion with phospholipases

The attention was further focused on the biochemical characterization of this protein, particularly of its lipid portion, and it was compared with some other GPI-linked molecules expressed on the surface of human erythrocytes. Many proteins of eukaryotic cells are anchored to membrane by covalent linkage to glycosyl phosphatidylinositol (GPI). Those proteins lack a transmembrane domain, have no cytoplasmic tail and are therefore located exclusively on the extracellular side of the plasma membrane. Glycosylated phosphoinositol anchored molecules are a structurally diverse family of biomolecules that includes: protozoan coat components, activation antigens, complement regulatory proteins, adhesion molecules, membrane-associated enzymes and many others glycoproteins. This type of cell membrane attachment implies that the protein moieties of this antigens are located outside the membrane, have potentially high lateral mobility and may be released from the cell by the action of glycosylphosphatidylinositol (GPI) specific phospholipase C. GPI anchors may be considered as a reasonably well defined class of the structures because important elements are conserved over a wide range of phylogeny, from protozoan parasites to mammals. This anchor contains a linear core glycan sequence of ethanolamine-PO₄-6 Man_{α}1-2Man_{α}1-6Man_{α}1-4GlcNH₂-_{α}1-6*myo*-inositol-1-PO4-lipid. The heterogeneity among different GPI anchored proteins occurs mainly in the lipid composition. The VSGs anchors contain only dimyristoyl phosphatidylinositol whereas GPIs of Leishmania promastigote surface proteinase and many mammalian GPI-anchored proteins, including those on erythrocyte, like acethylcholinesterase (AchE), decay-accelerating factor (DAF), and membrane inhibitor of reactive lysis (MIRL) contain almost exclusively 1-alkyl-2-acylinositol phospholipid, which appear to be characteristic of human anchors in general (Ratnoff et al., Clin. Exp. Immunol. 87 (1992), 415-421). In addition, some GPI anchors contain an additional fatty acid (palmitate) in a hydroxyester linkage to the inositol ring, which renders them resistant to the action of bacterial phosphatidylinositol specific phospholipase C (PI-PLC) and the GPI-specific phospholipase C (GPI-PLC) from *Trypanosoma brucei.* The basis of this phospholipase resistance was first described for human erythrocyte acetylcholinesterase, and subsequently for human erythrocyte decay accelerating factor. The presence of a substitution on the 2-position of inositol ring would explain the PI-PLC resistance of the palmitoylated anchors, since the bacterial PLC enzyme operates via nucleophilic attack of the phosphorus atom by the hydroxyl group at the 2-position of the inositol ring. This finding led to the suggestion that occupation of the myo-inositol 2-position hydroxy group by palmitate would automatically preclude the action of this enzyme. As shown for DAF and some other GPI-linked proteins they can exhibit differential inositol acetylation and this structural variation is regulated in a cell-specific fashion, but can also be protein dependent (Chen et al., PNAS USA 95 (1998), 9512-9517). The biological relevance of GPI-anchor structural variability among different blood cell types remains incompletely understood. The presence of additional fatty acid chain (acylation) in the hydrophobic domain probably improves the attachment of the molecule to the external leaflet of the membrane. The presence of this acyl-chain on the inositol ring in erythrocyte associated anchors, renders these structures resistant to cleavage by PI-PLC and less sensitive to PI-PLD cleavage when purified. The resistance to be released by phospholipases, either by blocking the lytic mechanism of a phosphatidyl-specific phospholipase C, or by retaining membrane association following cleavage of phosphatidic acid by anchor specific phospholipase D, may enhance the membrane stability of these proteins over time, consistent with the longer (120 day) life span in the circulation of erythrocytes, as compared with leukocytes.

The following experiments were primarily carried out in order to determine the primary structure and molecular parameters of the lipid structure of ACA and to compare these parameters with those of other GPI-linked proteins. The susceptibility of a protein to release from a membrane surface by bacterial phosphatidylinositol-specific phospholipase (PI-PLC) is the original indicator for the presence of a GPI anchor. However, as already mentioned above, some GPI anchors are resistant to hydrolysis by bacterial PI-PLC, like erythrocyte membrane proteins, and this resistance depends on additional acylations on inositol ring.

To investigate whether ACA is anchored in the membrane bilayer by covalent attachment of the C-terminal amino acid residue to a glycosylphosphatidylinositol, the specific hydrolysis of the phosphodiester bond of phosphatidylinositol was studied using different anchor degrading enzymes. First it was observed that the effect of PI-PLC on erythrocyte membrane protein fraction led to a disappearance of a very faint electrophoretical band which was later identified as the new human erythrocyte protein. Because of the very low abundance of this protein on the surface of erythrocytes, the lipid portion of this molecule was further analysed on the purified protein. Phospholipases C hydrolyses the phosphodiester linkage to produce 1,2-diacylglycerol, alkylacylglycerol and the phosphorylated polar head group, while the phospholipases D hydrolyses the phosphodiester linkage to produce a phosphorylated 1,2-diacyl glycerol or alkylacylglycerol and the polar head group with an exposed hydroxyl group. As shown in Figure 5A, there was no portion of the ACA protein which remained resistant to anchor degrading phospholipases. The native, intact protein exhibits slow migration, indicative of detergent micellar association.

The PI and GPI-PLC hydrolyzed protein of RBC ACA, exhibited more rapid migration, characteristic of a detergent-free hydrophilic species.

The ACA protein treated with glycosyl-phosphatidylinositol phospholipase D shows no changes in electrophoretic mobility, when compared to the native protein (Figure 5B). The lower electrophoretic mobility of GPI-PLD digested protein, when compared to the PI-PLC digested one, is due to the lack of a phosphate (negative charge) on the inositol ring of the degraded protein.

### Example 4: Presence of the cross-reacting determinant (CRD) epitope on ACA

A polyclonal antibody raised to the soluble form of the variant surface glycoprotein (sVSG) cross-reacts with other unrelated GPI-anchored proteins. This "cross-reacting determinant" (CRD) is only exposed when the protein is converted into a hydrophilic form by the action of either bacterial PI-PLC or eukaryotic GPI-PLC. In the case of soluble form of variant surface glycoprotein (sVSG), three overlapping epitopes are involved in this recognition, the inositol 1,2-cyclic phosphate generated on phospholipase C cleavage of the anchor, the non-acetylated glucosamine residue, and the variable galactose branch. It is also known, that regarding mammalian proteins, the major epitope involved in this recognition is the inositol 1,2-cyclic phosphate. Thus, the recognition of a protein, following phospholipase C cleavage by an anti-CRD antibody is the most powerful evidence for the presence of a GPI-anchor. In the next set of experiments it was sought to determine whether anti-CRD antibodies recognized a soluble form of isolated ACA protein. As demonstrated here, human erythrocytes ACA exhibit >99% PI-PLC sensitivity to the action of phospholipases C. Glycosyl-phosphatidylinositol phospholipase C digested protein was electrophoresed, and than subjected to Western-blotting using anti-CRD antibody (Figure 6). As positive control, the membrane form of variant surface glycoprotein (mVSG) was digested with the same anchor degrading phospholipases. As negative control, the native form of ACA and GPI-PLD cleaved protein were blotted simultaneously and labeled with anti-CRD antibody. The GPI-PLD cleaved molecules do not react with anti-CRD antibodies indicating that this enzyme leaves phosphodiacylglycerol and 1,6 myo-inositol as reaction products, destroying the CRD antigenic determinant by preventing the formation of D-myo-inositol 1,2-cyclic phosphate. The membrane form of ACA was also used as a negative control, as expected it was not recognized by anti-CRD antibodies.

### Example 5: Identification of the lipid substituents of the ACA anchor

PI-and GPI-PLC digested purified protein was further hydrolyzed with triacylglycerol acyl-hydrolase, giving the 1,2 diglyceride and fatty acid as first reaction products, followed by carboxylic ester hydrolase - an enzyme which accelerates the total hydrolysis by a specific cleavage of soluble glyceride, leaving glycerol and fatty acids as the final reaction products. Glycerol was then determined enzymatically, with GK, PK and LDH as auxiliary and indicatory enzymes. The scheme of all enzymatic reactions leading to total hydrolysis of lipid tail of ACA anchor, and enzymatic reactions for determinations of glycerol are outlined in Figure 1. The decrease in extinction at 340 nm, 334 nm and 365 nm due to oxidation of NADH is measured and released glycerol calculated in g/l. The amounts of released glycerol by hydrolysis with PI and GPI-phosphatidylinositol specific phospholipases followed by specific deacetylation were similar, indicating the same specific activity of both enzymes on ACA. (Table I).

**Table I:**

| **Determination of glycerol concentration** | | |
|---|---|---|
| **Release of glycerol** | | |
| | PI-PLC | GPI-PLC |
| 65 kDa | 0,045 g/l | 0,038 g/l |

The data show the amount of released glycerol after total hydrolysis of ACA lipid tail.

To further investigate the structure of the hydrophobic domain in red blood cells ACA particularly the fatty acids composition, the fragments generated were analyzed by total hydrolysis of the anchor. The photoactivated reagent [¹²⁵J] TID was used which was shown to partition strongly in favor of the lipid phase of membranes and the photogenerated carbene, labels the lipid groups of intrinsic membrane proteins in a highly selective manner. Samples of [¹²⁵J] TID labeled, purified proteins were subjected to hydrolysis with GPI- phospholipase C. The lipid products of these reactions were further hydrolyzed with highly specific lipases, triacylglycerol acylhydrolase and carboxylic-ester hydrolyse, as described in Experimental Procedures. The released radiolabeled fragments were extracted and analyzed by thin layer chromatography and autoradiography. Membrane form of variant surface glycoprotein (mfVSG) from *trypanosoma brucei* was [¹²⁵J] TID labeled and hydrolyzed in the same manner as ACA, and subjected to thin-layer chromatography as a control (Figure 7).

The major lipid species obtained by total hydrolysis of anchor of ACA exhibited the same mobility as labeled fatty acid tail of commercially available VSG, indicating the same 1,2-diacyl myristate structure, differing from other erythrocyte GPI-linked proteins which have alkyl-acyl glycerol and different fatty acids as a lipid tail of the molecule.

### Example 6: Generation of an ACA specific antibody and detection of its expression on erythrocytes

A purification method was established to obtain large amounts of protein necessary for immunization of 6 BALB/c mice, according to standard procedures. To **improve** the understanding of this novel molecule, first a mouse polyclonal antibody was produced and its specificity was tested with different samples of crude protein extract, purified protein, red cell membrane, and cell-free extracts. The specificity of the polyclonal antibody was tested in Western-blots, with various protein fractions. Ammonium sulfate protein extract obtained from erythrocyte membranes as described in Example 1, was dissolved in storage buffer, dialyzed against the same buffer and subjected to reducing SDS-PAGE. The homogeneously purified ACA protein and protein extracts obtained from erythrocyte ghosts and ghosts obtained from human erythroleukemia cell-line (K-562) were subjected to SDS-PAGE, and then transferred to nitrocellulose membranes. The polyclonal mouse serum specifically reacted with the 65 kD band corresponding to ACA protein (Figure 8). No reactions with other proteins were observed. The faint band of erythrocyte crude protein extract shows a high specificity of antibody to this novel antigen and a very low abundance of this molecule in the plasma membrane of red blood cells, compared to human blood malignant cells.

### Example 7: Expression of ACA on peripheral blood cells

To investigate the in vivo expression of ACA in various peripheral blood cells, flow cytometry and immunoblot analysis with the monoclonal Abs (AC1 and AB12) and polyclonal antibody AL1, directed against the ACA protein, were performed. The results are shown in Figure 9(a-f) and Figure 16(a,b). It was found that both monoclonal antibodies strongly react with granulocytes, monocytes and a small subpopulation of lymphocytes, indicating expression of ACA on those cells. The percentage of positive cells in each population obtained from the analysis of 20 healthy volunteers is shown in Figure 10.

### Example 8: GPI-anchoring of ACA

To investigate the form in which ACA is embedded in the membrane lipid bilayer, purified granulocytes and lymphocytes from normal volunteers, after treatment with phosphatidyl inositol-specific phospholipase C (PI-PLC), an enzyme which hydrolyses the GPI-anchor and removes GPI-linked proteins from the cell membrane, were analyzed. After one hour of exposure to PI-PLC, granulocytes became ACA negative, confirming the presence of the GPI-anchored protein. A representative histogram of PI-PLC treatment of granulocytes is shown in Figure 11b Additional negative control was performed on purified granulocytes with glycosylphosphatidylinositol phospholipase D (GPI-PLD). It is known that this enzyme reacts with GPI-linked proteins, even with those acetylated on an inositol ring - a chemical modification which renders many proteins resistant to action of PI-PLC. However, the enzyme cannot solubilize GPI-anchored proteins from intact membranes but only separates them from their anchor, if the proteins have been solubilized out of the membrane. Granulocytes from healthy donors were treated with GPI-PLD and as expected, ACA was not removed from the cell surface (Figure 11c). In control experiments, digestion with PI-PLC was performed on cultured human erythroleukemia (HEL) cell line and the expression of ACA and GPI-anchored DAF protein (CD55) were monitored. HEL cells become ACA- and CD55- negative, after the action of PI-PLC under the same reaction conditions as those used for granulocytes (Figure 11 d-f).

### Example 9: ACA is expressed on peripheral B cells

As shown in Figure 9 only a subpopulation of lymphocytes expresses ACA. In order to define the relevant subpopulation, expression of ACA was analyzed on purified peripheral B blood lymphocytes. In a first experiment, B-cells were isolated from healthy donors by rosetting. In one-color analysis with ACA-specific monoclonal antibodies more than 90% of isolated cells showed expression of the protein (Figure 12a). ACA was partly removed from the surface of those cells with PI-PLC under slightly different reaction conditions, as those used for digestion of granulocytes. A detergent, Triton X-100 was added to the enzyme solution, to increase its specific activity. Staining by ACA antibodies was markedly reduced, suggesting that this antigen is also phosphatidylinositol (PI)-linked (Figure 12b). However, treatment with higher concentrations of PI-PLC did not give a further decrease in binding of ACA antibodies suggesting that not all molecules are sensitive to this enzyme (Figure 12b). Two-color analysis of purified B lymphocyte shows that all CD 19 and CD20 positive cells were ACA positive (Figure 12 c-e), peripheral blood lymphocytes shows that about 10-15% of cells coexpresss CD19, CD20, and ACA corresponding to the B lymphocyte subpopulation (Figure 12 f-h).

### Example 10: Expression of ACA on EBV-transformed normal and paroxysmal nocturnal haemoglobinuria (PNH) B lymphocytes

The expression of ACA in EBV-transformed normal and PNH patient B lymphocytes was further analyzed. Paroxysmal nocturnal haemoglobinuria is an acquired haematological disorder characterized by complement-mediated haemolitic anaemia caused by deficient GPI anchored proteins. In one color flow cytometry, EBV-transformed PNH B lymphocytes show significantly reduced fluorescence (Figure 13b) using anti-ACA antibody, when compared with EBV-transformed B lymphocytes obtained from a healthy volunteer (Figure 13a).

### Example 11: ACA is not expressed on T-cells

Expression of ACA on T-cells was analyzed by double-staining of purified peripheral blood T lymphocytes with ACA-specific antibodies (Figure 14). T-cells were isolated from peripheral blood as described in Material and Methods and double stained with typical T-cell markers (CD3, CD4 and CD8). Of purified T lymphocytes more than 95% were ACA-negative (Figure 14 a-d). Additionally, two-color analysis of PBMC confirmed the previous data. Cells expressing typical T-cell markers like CD2 and CD5 were ACA negative (Figure 14e-h). Moreover, even the γδ T-cell subpopulation was ACA-negative (Figure 14f). GPI-positive and GPI-negative T-cell clones obtained from PNH patients were analyzed by two-color flow cytometry. Even cells expressing the GPI-linked CD48 antigen were completely ACA-negative. Representative histograms are shown in Figure 15 a,b,c. Additionally, various cell-lines derived from malignancies of T-cell origin (MOLT 4, CEM, JURKAT J6) were analyzed. None of them showed surface expression of ACA (Figure 15 d-f). The findings led us to conclude that ACA is expressed exclusively on B-cells.

### Example 12: Detection of ACA in solubilized membranes of erythrocytes, granulocytes, and B-cells

Solubilized ACA obtained from various peripheral blood cells was electrophoretically separated and transferred to nitrocellulose. The filters were then probed with anti-ACA antibodies. The antibodies recognized a 65 kD protein corresponding to ACA in solubilized granulocytes and B-cells (Figure 16a). Moreover, the ACA protein was immunodetected in supernatants of granulocyte cultures after- but not before treatment with PI-PLC (Figure 16b). In contrast to the negative results obtained by flow cytometry regarding the expression of ACA on erythrocytes - a finding reflecting the very low abundance of the molecule on these cells, immunoblotting with polyclonal and monoclonal antibodies clearly shows the presence of ACA on erythrocytes (Figure 16).

### Example 13: ACA is overexpressed in melanoma and some leukemia cells

To elucidate the possible role of ACA in specific signal transduction pathways during carcinogenesis, a large series of benign and malignant cell lesions and their metastases were studied for the expression and distribution of ACA.

### Results

### (A) Distribution of ACA in normal skin

### Weak ACA-expression in resting melanocytes

ACA is expressed in normal skin in melanocytes scattered through the basal layer. Langerhans cells (derived from monocytes!) in supra basal layer are stained, but to a lesser extent (Figure 17 a, b).

### Strong ACA-expression in dividing melanocytes

Melanocytes are stained heterogeneously with monoclonal antibody to ACA. The highest expression of ACA was found when the cells divided, i.e. progressed from G2 phase to mitosis (Figure 18 a, b).

### No ACA-expression on keratinocytes

Primary human epidermal keratinocytes and keratinocyte cell line (HaCaT cells)(a gift of Prof. Fusenig, German Cancer Research Center) show no expression of ACA in immunocytological staining (Figure 19 a, b) or Western Blot (Figure 19c).

### (B) Expression of ACA in skin neoplasia

### Strong ACA-expression in dividing melanocytes of Congenital Nevus

Congenital Nevus melanocytes in basal epidermal layer and above react strongly with antibodies to ACA. One third of large congenital nevi expressed ACA in small clusters of heavily pigmented cells, corresponding to so-called proliferative nodules. The same cell type in dermal layer (predominantly non-dividing melanocytes!) was not stained. These results confirm the previous finding about preferential expression of ACA in dividing cells and indicate that cells with metastatic potential have the highest expression of ACA (Figure 20).

### Strong ACA-expression in melanoma cells

Expression of ACA is highly upregulated in primary metastatic melanoma (Figure 21a, b). Therefore, antibodies to ACA can be used for detection of melanoma metastasis (Figure 22 a, b).

### ACA is not expressed in basalioma and spinalioma

Expression of ACA is specific for melanocytic and melanoma lesions. Other types of skin tumors like basalioma and spinalioma are ACA negative (Figure 23 a, b).

### High ACA-expression in melanoma is confirmed by immunoblotting

Immunoblot analysis of homogenized normal skin and melanoma tumor tissues with monoclonal antibodies to ACA confirm the specificity of antibodies and elevated expression of this protein in melanoma when compared to normal tissues (Figure 24).

### (C) Expression of ACA in other tumors

Immunoblot analysis of homogenized fresh tumor tissues shows expression of ACA antigen in renal, lung, breast, colon, gastric cancer, melanoma and myeloma (Figure 25).

### (D) Expression of ACA in hematopoietic cells

### ACA is expressed in normal human progenitor cells

A purified bone marrow progenitor cell preparation was analyzed by one or two-color flow cytometry as described in Example 1 regarding ACA co-expression with various cell markers. Figure Figure 26 b-d analyzes the progenitor cell population used in this study (co-expression of CD34/CD38, CD34/CD90, CD34/CD117). Figures 26e-h show co-expression of ACA on CD34/CD38-, CD34/CD90-, CD34/CD117-, CD34/HLA-DR-positive cells. Figures 26j-1 show co-expression of ACA with the following markers: CD13 (myeloid marker) (Fig. 26j), CD33 (panmyeloid marker) (Fig. 26k), CD34 (hematopoietic precursor cell marker) (Fig. 261). Figures 26a,i represent negative controls.

### ACA is expressed in leukemia cells

One-color flow cytometry histograms show expression of ACA on human erythroleukemia (HEL) ( Fig. 27a), human promyelocytic leukemia (HL-60) (Fig. b), and human chronic myelogenous leukemia (K-562) (Figure 27 c). Immunoblot analysis with anti-ACA antibodies of cell-free extracts of human leukemia cell lines confirms ACA-expression and shows that ACA has the same molecular size as originally described for erythrocytes (Figure 28).

### Expression of ACA on granulocytes from paroxysmal nocturna hemoglobinuria (PNH) patients

ACA is expressed on PNH granulocytes but not on PNH B lymphocytes, although normal B cells express the protein. PNH is a clonal stem cell disorder characterized by complement-mediated hemolysis, deficient haematopoesis and occasionally, leukemia. The biochemical disturbance in PNH involves the defective synthesis of GPI anchors. As a result of this defect, affected cells are missing all surface proteins that use GPI-linkage including proteins involved in complement regulation, immunologic receptors, enzymes and several with unknown function.

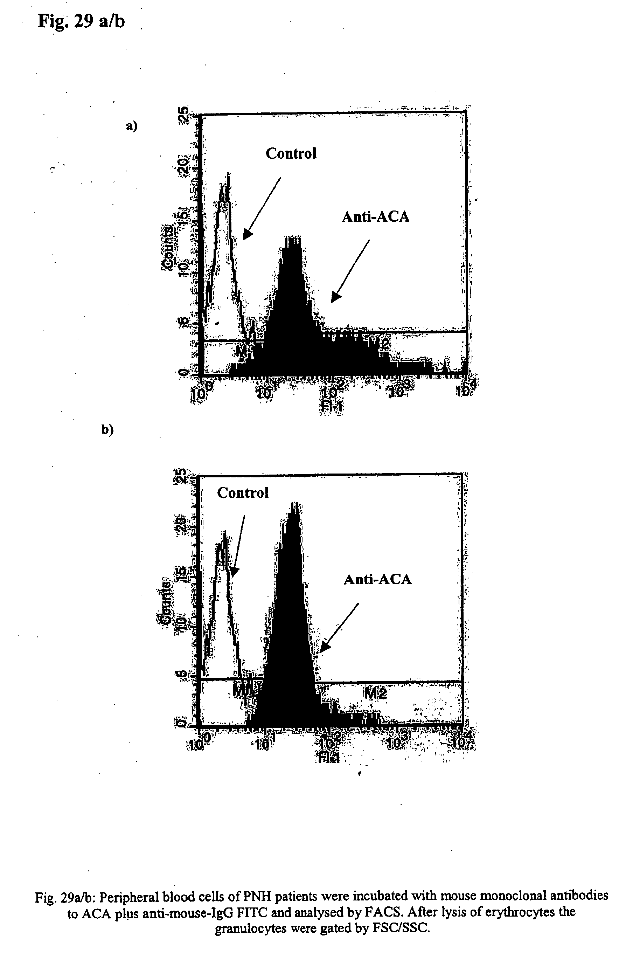

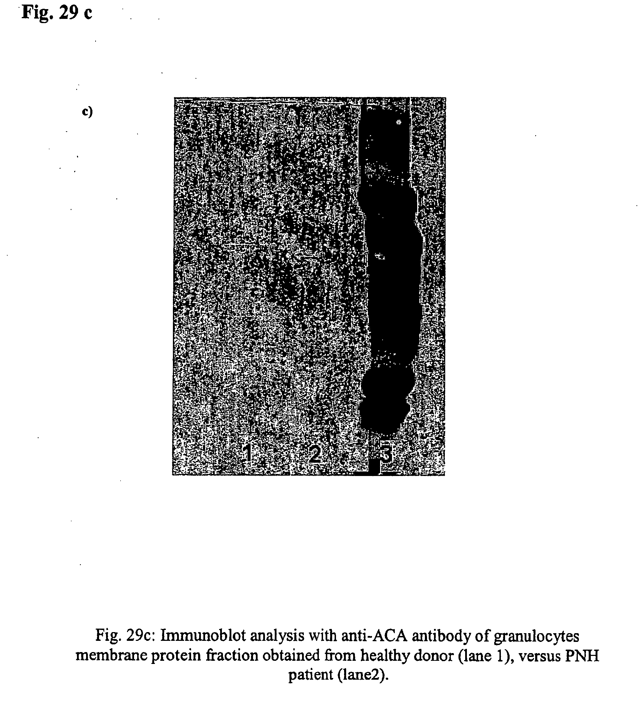

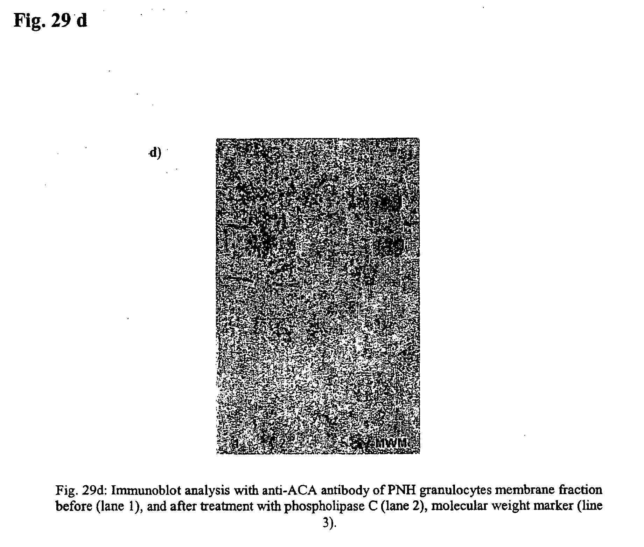

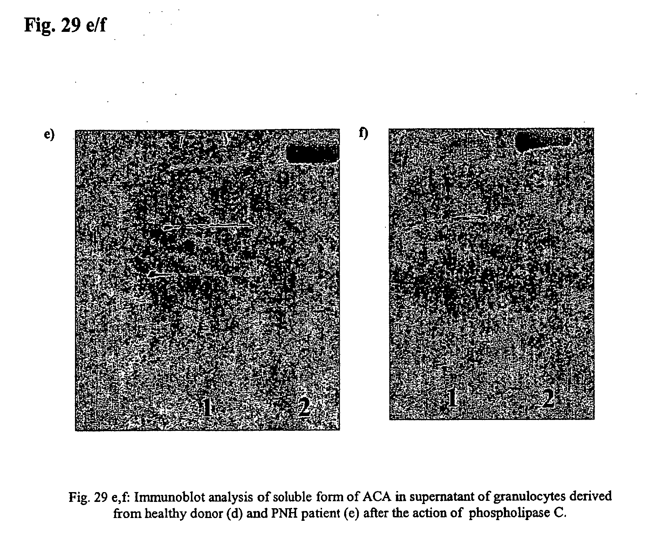

The molecular defect found in PNH is due to one or more acquired mutations in PIG-A, an X-linked gene involved in the first step of GPI anchor biosynthesis. An unusual expression pattern of ACA was found in PNH blood cells. EBV-transformed B-lymphocytes of PNH patients have drastically reduced ACA expression, while PNH granulocytes show normal surface expression of this antigen (Figure 29).

The Representative histograms show:
Normal surface expression of ACA on PNH granulocytes (Fig. 29a,b).
Immunoblot analysis with anti-ACA antibody of granulocytes membrane protein fraction obtained from healthy donor (lane 1) versus PNH patient (lane 2)(Figure 29c).
Immunoblot analysis with anti-ACA antibody of PNH granulocytes membrane protein fraction, before and after treatment with phospholipase C (Figure 29d).
The soluble form of ACA in supernatant of granulocytes derived from healthy persons (Figure 29e) and PNH patients (Figure 29f).

The presence of ACA on PNH granulocytes could probably explain some properties of abnormal PNH granulocytes not related with PIG-A mutation. It has long been observed that abnormal PNH cells achieve clonal dominance within the bone marrow and peripheral blood. In many patients with PNH, more than 80% of their circulating granulocytes and erythrocytes are GPI deficient, suggesting that the abnormal PNH clone has a growth advantage over normal progenitors. PNH cells are also relatively resistant to apoptosis and this feature appears to be the principle mechanism by which PNH cells maintain a growth advantage over normal progenitors and could play a role in the propensity of the disease to transform into more aggressive hematologic disorders. Unregulated clonal growth may occur from signals that enhance cell proliferation or alternatively from signals that block apoptosis and enhance cell survival. The presence of ACA on PNH granulocytes may confer this proliferative advantage to the mutated hematopoetic stem cell and could play a role in a developing of leukemia.

How could ACA be expressed on the surface of PNH granulocytes in spite of PIG-A mutation? It has already been demonstrated the unique potential for GPI-anchored proteins to transfer from one cell membrane to another. The GPI-anchored proteins were shown to transfer intact and functional. Implications of this phenomenon exist in many areas including disease transmission (prions), cell protection (endothelial cells) or protein engineering of cell surfaces, which is a potentially powerful technology through which the surface protein composition of cells can be manipulated without gene transfer. This observation demonstrates the potential for GPI-linked proteins to be "painted" onto cells which otherwise may be incapable of expressing exogenous proteins. It is possible that also ACA "paint" into PIG-A mutated PNH cell from another cell (probably stem cell!) capable of synthesizing anchors and according to the already suggested functions of ACA, could be responsible for all abnormalities already described for PNH granulocytes.

Expression of ACA in normal skin and melanoma shows the same phenomenon. In normal skin ACA is expressed in melanocytes scattered through stratum basale and to a lesser extent in suprabasal layer. Expected expression on Langerhans cells (derived from monocytes!) was found but also a discrete reaction ("fading out") of anti-ACA antibodies with keratinocytes. Keratinocytes in culture show no reaction with anti-ACA antibodies. Immunoblot analysis of keratinocytes with anti-ACA antibody was also negative. These data indicate that ACA is probably moving from another cells and paint onto keratinocytes. Frozen sections of primary melanoma and melanoma metastasis stained with anti-ACA antibodies show very strong expression of this protein and the same "fading out" phenomenon seen also in normal skin.

Congenital Nevus shows strong expression of ACA in melanocytes beyond the basal membrane, again with this "fading out" phenomenon. All these data strongly suggest that a general mechanism exists that enable ACA to move between the cells. Taking into account that ACA has an anchor with diacyl myristat (DAG) as a lipid tail, molecule known as a potent activator of some protein kinases and stimulator of DNA synthesis, it is possible that ACA is moving from malignant melanoma cells (probably with some modification on anchor!) incorporate into a new melanocytes contributing to its uncontrolled growth. Extremely short time for the developing of metastasis in skin could be explained with this ability of ACA protein.

## Claims

1. A surface glycoprotein comprising the following features:
(a) it is GPI-anchored on the cell surface;
(b) it can be removed from the cell membrane by treatment with PI-PLC; and
(c) its GPI-anchor is **characterized by** a non-acetylated inositol ring and diacyl glycerol as lipid tail of the anchor.

2. The surface glycoprotein of claim 1 which is the surface glycoprotein ACA **characterized by** the following additional features:
(d) it has an isoelectric point of pH 5.5;
(e) it is present on progenitor cells, granulocytes, monocytes, B-cells (but not T-cells), melanocytes, and other cells; and
(f) it is preferentially expressed during cell division and in tumor cells;
or a salt, functional derivative or active fraction thereof.

3. The surface glycoprotein ACA of claim 2, obtainable from human blood by
(a) isolating and lysing cells;
(b) isolating, disrupting and pelleting the hemoglobin free membrane of said cells;
(c) repeated salting out of the resuspended membranes with ammonium sulfate (70%; 40% saturation);
(d) subjecting the proteins precipitated in step (c) to preparative SDS-PAGE under reducing conditions; and
(e) isolating the gel band of the protein.

4. The surface glycoprotein ACA of claim 2 or 3 having a molecular weight of about 65 kD when analyzed by SDS PAGE under reducing conditions.

5. The surface glycoprotein ACA of any one of claims 2 to 4 which contains at least one of the following amino acid sequences:
(a) D-L-V-P-L-E-D-K-V-T-I-L-G-M-T-A;
(b) K-L-A-L-S-A-D-D-P-G-F-H-N-F-S-H-Q-R-Q-T;
(c) D-Q-Q-T-T-S-H-S-S;
(d) V-L-E-I-M-L-P;
(e) F-Q-D-E-S-E-A-N-K;
(f) M-K-Y-V-N-F-K-F-Y-F;
(g) N-L-D-F-M-T-W-G-V-T-K-V-T-Y-I-G-Q-P-T-G-G;
(h) L-L-M-D-N-N-E-A-V-H;
(i) F-D-Q-A-W-A-D-T-A-H-T-W;
(j) K-L-D-D-I-Q-K-D-M-Y-S-Q-Q-D-T; or
(k) G-V-W-I-M-K-N-Q-I-T.

6. The surface glycoprotein ACA of any one of claims 2 to 5 which is isolated from blood cells.

7. A process for the isolation of a surface glycoprotein ACA which comprises:
a) isolating and lysing cells from human blood;
(b) isolating, disrupting and pelleting the hemoglobin free membrane of said cells;
(c) repeated salting out of the resuspended membranes with ammonium sulfate (70%; 40% saturation);
(d) subjecting the proteins precipitated in step (c) to preparative SDS-PAGE under reducing conditions; and
(e) isolating the gel band of a 65 kD protein.

8. The surface glycoprotein ACA produced by the process of claim 7.

9. The surface glycoprotein of any one of claims 1 to 6 which is a recombinant protein.

10. The surface glycoprotein of claim 9 which is produced in a mammalian cell.

11. A nucleic acid molecule comprising a nucleotide sequence encoding the surface glycoprotein ACA or a functional derivative or fragment thereof of any one of claims 2 to 7, wherein said surface glycoprotein ACA contains at least one of the following amino acid sequences:
(a) D-L-V-P-L-E-D-K-V-T-I-L-G-M-T-A;
(b) K-L-A-L-S-A-D-D-P-G-F-H-N-F-S-H-Q-R-Q-T;
(c) D-Q-Q-T-T-S-H-S-S;
(d) V-L-E-I-M-L-P;
(e) F-Q-D-E-S-E-A-N-K;
(f) M-K-Y-V-N-F-K-F-Y-F;
(g) N-L-D-F-M-T-W-G-V-T-K-V-T-Y-I-G-Q-P-T-G-G;
(h) L-L-M-D-N-N-E-A-V-H;
(i) F-D-Q-A-W-A-D-T-A-H-T-W;
(j) K-L-D-D-I-Q-K-D-M-Y-S-Q-Q-D-T; or
(k) G-V-W-I-M-K-N-Q-I-T.

12. The nucleic acid molecule of claim 11 wherein the nucleotide sequence is a genomic DNA sequence or a cDNA sequence.

13. An expression vector comprising the nucleic acid molecule of claim 11 or 12.

14. A host cell transformed with the expression vector of claim 13.

15. The host cell of claim 14 which is a mammalian host cell.

16. A process for producing a surface glycoprotein ACA comprising the steps of:
(a) culturing a transformed host cell according to claim 14 or 15 in a suitable culture medium; and
(b) isolating the protein form the cells or the culture medium.

17. An antibody to the surface glycoprotein according to any one of claims 1 to 6 or 8 to 10.

18. The antibody of claim 17 which is a monoclonal antibody.

19. A method for the diagnosis of a tumor associated with overexpression of ACA or a predisposition for such a tumor which comprises
(a) contacting a target sample with a compound which is capable of specifically binding (i) to the surface glycoprotein ACA according to any one of claims 2 to 6 or 8 to 10 or (ii) an mRNA transcribed from the nucleic acid molecule of claim 11 or 12 and determining the level of ACA or ACA mRNA; and
(b) comparing the level of ACA protein or ACA-mRNA of the sample determined by use of the compound of step (a) with a control sample obtained from a healthy individual, wherein an elevated level of the surface glycoprotein ACA or the corresponding mRNA is indicative for a tumor or a predisposition for such a tumor.

20. The method of claim 19, wherein the compound is an antibody of claim 17 or 18 or an oligonucleotide which is capable of hybridizing to an mRNA transcribed from the nucleic acid molecule of claim 11 or 12.

21. A pharmaceutical composition containing a compound capable of reducing or eliminating (a) the expression of the nucleic acid sequence encoding the surface glycoprotein ACA and/or (b) the biological activity of ACA.

22. Use of a compound capable of reducing or eliminating (a) the expression of the nucleic acid sequence encoding the surface glycoprotein ACA and/or (b) the biological activity of ACA for the preparation of a pharmaceutical for the treatment of cancer or prophylaxis.

23. The method of claim 19 or 20 or the use according to claim 22, wherein the cancer is a melanoma, leukemia, renal cancer, lung cancer, breast cancer, colon cancer, gastric cancer, or any other form of cancer.

24. A kit containing the antibody of claim 17 or 18 or an oligonucleotide which is capable of hybridizing to an mRNA transcribed from the nucleic acid molecule of claim 11 or 12.


SEQUENCE LISTING

<110>  Becker-Kojic, Zorica

<120>  GPI anchored glycoprotein ACA as a novel tumor marker

<130>  B 1841EP

<140>  EP 02017524.6
<141>  2002-08-02

<160>  11

<170>  PatentIn version 3.1

<210>  1
<211>  16
<212>  PRT
<213>  Artificial

<220>
<223>  fragment of the glycoprotein ACA

<400>  1

Asp Leu Val Pro Leu Glu Asp Lys Val Thr Ile Leu Gly Met Thr Ala
1               5                   10                  15


<210>  2
<211>  20
<212>  PRT
<213>  Artificial

<220>
<223>  fragment of the glycoprotein ACA

<400>  2

Lys Leu Ala Leu Ser Ala Asp Asp Pro Gly Phe His Asn Phe Ser His
1               5                   10                  15


Gln Arg Gln Thr
            20


<210>  3
<211>  9
<212>  PRT
<213>  Artificial

<220>
<223>  fragment of the surface glycoprotein ACA

<400>  3

Asp Gln Gln Thr Thr Ser His Ser Ser
1               5


<210>  4
<211>  7
<212>  PRT
<213>  Artificial

<220>
<223>  fragment of the surface glycoprotein ACA

<400>  4

Val Leu Glu Ile Met Leu Pro
1               5


<210>  5
<211>  9
<212>  PRT
<213>  Artificial

<220>
<223>  fragment of the surface glycoprotein ACA

<400>  5

Phe Gln Asp Glu Ser Glu Ala Asn Lys
1               5


<210>  6
<211>  10
<212>  PRT
<213>  Artificial

<220>
<223>  fragment of the surface glycoprotein ACA

<400>  6

Met Lys Tyr Val Asn Phe Lys Phe Tyr Phe
1               5                   10


<210>  7

<211>  21
<212>  PRT
<213>  Artificial

<220>
<223>  fragment of the surface glycoprotein ACA

<400>  7

Asn Leu Asp Phe Met Thr Trp Gly Val Thr Lys Val Thr Tyr Ile Gly
1               5                   10                  15

Gln Pro Thr Gly Gly
            20

<210>  8
<211>  10
<212>  PRT
<213>  Artificial

<220>
<223>  fragment of the surface glycoprotein ACA

<400>  8

Leu Leu Met Asp Asn Asn Glu Ala Val His
1               5                   10

<210>  9
<211>  12
<212>  PRT
<213>  Artificial

<220>
<223>  fragment of the surface glycoprotein ACA

<400>  9

Phe Asp Gln Ala Trp Ala Asp Thr Ala His Thr Trp
1               5                   10

<210>  10
<211>  15
<212>  PRT
<213>  Artificial

<220>
<223>  fragment of the surface glycoprotein ACA

<400>  10

Lys Leu Asp Asp Ile Gln Lys Asp Met Tyr Ser Gln Gln Asp Thr
1               5                   10                  15


<210>  11
<211>  10
<212>  PRT
<213>  Artificial

<220>
<223>  fragment of the surface glycoprotein ACA

<400>  11

Gly Val Trp Ile Met Lys Asn Gln Ile Thr
1               5                   10